# EUROPEAN PATENT SPECIFICATION

(11) **EP 2 761 520 B1**
(45) Date of publication and mention of the grant of the patent: **13.05.2020**
(21) Application number: 12836365.2
(22) Date of filing: 26.09.2012
(51) Int. Cl.: G16B 20/20, G16B 50/30

(54) **DIAGNOSTIC METHOD AND SYSTEM FOR GENETIC DISEASE SEARCH BASED ON THE PHENOTYPE AND THE GENOME OF A HUMAN SUBJECT**
DIAGNOSTISCHES VERFAHREN UND SYSTEM ZUR SUCHE NACH GENETISCHEN KRANKHEITEN AUF DER BASIS DES PHENOTYPS UND DES GENOMS EINER PERSON
PROCÉDÉ ET SYSTÈME DIAGNOSTIQUE POUR LA RECHERCHE DES MALADIES GÉNÉTIQUES SUR LA BASE DU PHÉNOTYPE ET DU GÉNOME D'UN SUJET HUMAIN

(30) Priority: 26.09.2011 US 201161539020 P; 21.11.2011 US 201161562110 P; 04.07.2012 US 201261668021 P
(43) Date of publication of application: 06.08.2014
(73) Proprietor: Trakadis, John, Montreal, Québec H3P 2K1 (CA)
(72) Inventor: Trakadis, John, Montreal, Québec H3P 2K1 (CA)
(74) Representative: Capitán García, Maria Nuria
(86) International application number: PCT/CA2012/000881
(87) International publication number: WO 2013/044354

(56) References cited:
- WO-A1-2010/039526
- GB-A- 2 444 410
- JAMISON D FERAMISCO ET AL: "Phenotypic and Genotypic Analyses of Genetic Skin Disease through the Online Mendelian Inheritance in Man (OMIM) Database", JOURNAL OF INVESTIGATIVE DERMATOLOGY, vol. 129, no. 11, 18 June 2009 (2009-06-18), pages 2628-2636, XP055173407, ISSN: 0022-202X, DOI: 10.1038/jid.2009.108
- R A GEORGE ET AL: "General mutation databases: analysis and review", JOURNAL OF MEDICAL GENETICS, vol. 45, no. 2, 24 September 2007 (2007-09-24), pages 65-70, XP055172704, DOI: 10.1136/jmg.2007.052639
- KANTARCIOGLU M ET AL: "A Cryptographic Approach to Securely Share and Query Genomic Sequences", IEEE TRANSACTIONS ON INFORMATION TECHNOLOGY IN BIOMEDICINE, IEEE SERVICE CENTER, LOS ALAMITOS, CA, US, vol. 12, no. 5, 1 September 2008 (2008-09-01), pages 606-617, XP011345491, ISSN: 1089-7771, DOI: 10.1109/TITB.2007.908465
- YANNIS J TRAKADIS: "Patient-controlled encrypted genomic data: an approach to advance clinical genomics", BMC MEDICAL GENOMICS, BIOMED CENTRAL LTD, LONDON UK, vol. 5, no. 1, 20 July 2012 (2012-07-20), page 31, XP021117126, ISSN: 1755-8794, DOI: 10.1186/1755-8794-5-31
- TRAKADIS YJ.: 'Patient-controlled encrypted genomic data: an approach to advance clinical genomics' BMC MED GENOMICS vol. 5, no. 1, 20 July 2012, XP021117126
- TIFFIN ET AL.: 'Prioritization of candidate disease genes for metabolic syndrome by computational analysis of its defining phenotypes' PHYSIOL. GENOMICS vol. 35, no. 1, 08 July 2008, pages 55 - 64, XP055148913
- LINGHU ET AL.: 'Genome-wide prioritization of disease genes and identification of disease-disease associations from an integrated human functional linkage network' GENOME BIOLOGY 2009, 10:R91 vol. 10, no. 9, 03 September 2009, XP021061453
- XIONG: 'PGMapper: a web-based tool linking phenotype to genes' BIOINTORMATICS vol. 24, no. 7, 18 January 2008, pages 1011 - 1013, XP055148914

## Description

### TECHNICAL FIELD

The present disclosure relates to searches related to the genome. More particularly, but not exclusively, the present disclosure relates a method and system for genetic disease/trait searches based on the phenotype and the genome of a human subject. More specifically, but not exclusively, the present disclosure relates a method for individualized genome based phenotype online searches and a system for such searches.

### BACKGROUND

In current clinical practice, in order to identify the genetic variant responsible for a patient's disease, first there is recognition of a more or less specific phenotype followed by molecular testing of a series of relevant genetic loci, individually or in small sets.

There exist a variety of online search engines using databases which aim to catalogue all the known diseases with a strong genetic component (e.g. mendelian genetic syndromes) and link at least some of these diseases to the relevant genes in the human genome. These services provide resources for research and for clinical genetic analysis of catalogued genes. Several of these websites are free and constantly updated.

Typically, to aid in the diagnosis of rare genetic syndromes, the physician performs a database search using patient-specific information derived from the medical evaluation performed in clinic. The physician then verifies the search-results and clinically prioritizes the associated diseases that the physician considers more likely. The physician then decides which genetic test to request based on the physical exam of the patient, prior lab tests and the like. Finally, samples (usually a blood sample) are sent for a molecular/genetic test to identify the mutation and confirm or rule out the genetic disease most highly suspected. Clinical genetic tests are often very expensive so usually only one gene, or less frequently a group of pre-selected genes usually responsible for one disease, is analyzed at a given time. If the first test is negative, sequential testing of the genes responsible for the other, less likely but still clinically suspected, genetic diseases usually follows.

Once a genetic change is identified the physician can use different databases and software, many of which are freely available, to determine if the genetic change identified is a normal variant unrelated to the patient's clinical features or a pathogenic (i.e. disease-causing) mutation.

The previously mentioned websites, which are used to aid in the diagnosis of rare genetic syndromes, list the genes for known syndromes but the search engine is only operated by searching for specific clinical characteristics (phenotype) keywords. Therefore, the searches are only taking into consideration the clinical information of each patient and not integrating the patient's genomic data. This leads to a less effective search which exacerbates the high costs of sequential clinical genetic tests.

New genomic technologies allow us to carry multiple genetic tests simultaneously to the extent that it is now possible to screen all genes for mutations as a "single" test. Methods like exome or genome sequencing do not target a single gene but the entire genome. At present entire exome/genome sequencing is mainly limited to research. It is typically used to identify the mutant gene which could explain a specific disease (phenotype) at hand which has not-yet been genetically characterized. Commercially, genome sequencing has been promoted as an educational tool which can estimate the risk of a person developing any genetic disease/trait but the clinical utility of these services is minimal if any.

The price of DNA sequencing drops faster than our understanding of how to interpret the results from multiple genetic tests improves. There are several limitations currently preventing us from using exome/genome sequencing as a clinical test. For example, the current percentage of false negative and false positive results of exome/genome sequencing prevents physicians from using it as a clinical diagnostic test. The cost for exome/genome sequencing as a wide-spread first-tier test would be prohibitively high if sequencing coverage increased enough to decrease the false positive and false negative results in order to allow it to be useful as a clinical diagnostic test. Another challenge we face is the extent to which it is possible to obtain meaningful patient informed-consent to large-scale genomic analysis. Genomic approaches increase the chance of uncovering clinically useful results, such as a mutation in a gene causing severe disease, unrelated to the initial clinical focus. For example, one could find a gene predisposing to an untreatable progressive neurological disease when performing exome sequencing to evaluate a child for an unrelated congenital heart disease. Another example would be identifying mutations in a gene predisposing to breast cancer (e.g. BRCA1 or BRCA2) when evaluating a child for unrelated short stature. The clinical interpretation and management of such a finding can often be problematic since one cannot distinguish people who will develop the disease from those who will not. A great proportion of such incidental findings involve probabilities, not certainties. Currently, genetic risk assessment is restricted to individuals at increased risk based on family history or clinical presentation, which may be indirectly ensuring the necessary genomic (or environmental) background for the pathogenic role of a variant. Recent reports suggest that on average, each person is heterozygous for approximately 50-100 variants classified by the Human Gene Mutation Database (HGMD) as causing inherited disorders and of approximately 250 to 300 loss of function variants in annotated genes. The degree of certainty with which we can we predict the impact of even known pathogenic variants when they are identified as incidental findings is not clear. Moreover, many of the generated findings are of uncertain clinical significance, such as mutations with incomplete penetrance, mutations in novel genes with unknown function, and genetic variants that are responsible for very small increases in disease risk. Variants associated with relative risks of diseases on the order of 1.5 or lower are very challenging to conceptualize and they can generate emotional distress about disease risk even among healthy individuals. The potential benefits for individuals need to be weighed against the potential harm and the individuals' right not to know. Respecting the patient's autonomy becomes a big issue since the patient or their family may not have wished to learn this information which may have personal, social implications (often negative) but is not always clinically useful. Finally, responsibly managing the very large amounts of genetic information produced is another key challenge to overcome, particularly in the context of the continuously evolving nature of interpretation and shortage of specialists with the appropriate expertise to interpret these data and to provide follow-up information and clinical care.

It appears that, in the light of rapid developments in genomic technologies, medical genetics is shifting from the present "phenotype-first" medical model to a "data-first" model which leads to multiple complexities.

There thus remains a need for improved online searches, especially using the "phenotype-based" and particularly the "phenotype-first" medical model, to better match gene mutations to the disease inflicting the patient.

### OBJECTS

An object of the present disclosure is to provide a method for an online phenotype search based on the genome of a human subject.

An object of the present disclosure is to provide a method for a phenotype search based on the genome of a human subject.

An object of the present disclosure is to provide a method for an online genetic trait search based on the phenotype and the genome of a human subject.

An object of the present disclosure is to provide a method for a genetic trait search based on the phenotype and the genome of a human subject.

An object of the present disclosure is to provide a method for an online genetic disease search based on the phenotype and the genome of a human subject.

An object of the present disclosure is to provide a method for a genetic disease search based on the phenotype and the genome of a human subject.

An object of the present disclosure is to provide a method for individualized genome based phenotype searches.

An object of the present disclosure is to provide a method for individualized mutation based phenotype searches

An object of the present disclosure is to provide a method of ranking a plurality of possible genetic diseases of a human subject related to the clinically assessed phenotypes of this human subject using a database of genetic diseases linked at least in part to at least one relevant region in the human genome and at least in part to at least one phenotypic characteristic.

An object of the present disclosure is to provide a method of ranking a plurality of possible genetic traits of a human subject related to the assessed phenotypes of this human subject using a database of genetic traits linked at least in part to at least one relevant region in the human genome and at least in part to at least one phenotypic characteristic.

An object of the present disclosure is to provide a method for a genetic condition search based on the phenotype and the encrypted genome of a human subject in conjunction with a database containing phenotypic and encrypted genomic information about other human subjects.

An object of the present disclosure is to provide a method for an online search for the genotype causing a specific genetic condition based on the phenotype and the encrypted genome of a human subject in conjunction with a database containing phenotypic and encrypted genomic information about other human subjects.

An object of the present disclosure is to provide a method for a search for the genotype causing a specific genetic condition based on the phenotype and the encrypted genome of a human subject in conjunction with a database containing phenotypic and encrypted genomic information about other human subjects.

An object of the present disclosure is to provide a method of ranking a plurality of possible genetic causes for a genetic condition of a human subject related to the assessed phenotypes of this human subject using a database containing genomic and phenotypic information of other human subjects

An object of the present disclosure is to provide a system for as online phenotype search based on the genome of a human subject

An object of the present disclosure is to provide a system for a phenotype search based on the genome of a human subject.

An object of the present disclosure is to provide a system for an online genetic trait search based on the phenotype and the genome of a human subject.

An object of the present disclosure is to provide a system for a genetic trait search based on the phenotype and the genome of a human subject.

An object of the present disclosure is to provide a system for an online genetic disease search based on the phenotype and the genome of a human subject.

An object of the present disclosure is to provide a system for a genetic disease search based on the phenotype and the genome of a human subject.

An object of the present disclosure is to provide a system for an individualized genome based phenotype searches.

An object of the present disclosure is to provide a system for individualized mutation based phenotype searches.

An object of the present disclosure is to provide a system of ranking a plurality of possible genetic diseases of a human subject related to the clinically assessed phenotypes of this human subject.

An object of the present disclosure is to provide a system of ranking a plurality of possible genetic traits of a human subject related to the assessed phenotypes of this human subject.

An object of the present disclosure is to provide a system for a genetic trait search based on the phenotype and the genome of a human subject in conjunction with a database containing phenotypic and genomic information about other human subjects.

An object of the present disclosure is to provide a system for an online search for the genotype causing a specific genetic trait based on the phenotype and the genome of a human subject in conjunction with a database containing phenotypic and genomic information about other human subjects.

An object of the present disclosure is to provide a system for a search for the genotype causing a specific genetic trait based on the phenotype and the genome of a human subject in conjunction with a database containing phenotypic and genomic information about other human subjects.

An object of the present disclosure is to provide a system for individualized phenotype based mutation searches

An object of the present disclosure is to provide a system of ranking a plurality of possible genetic causes for a genetic trait of a human subject related to the assessed phenotypes of this human subject using a database containing genomic and phenotypic information of other human subjects.

### SUMMARY

In accordance with an aspect of the disclosure, there is provided a computer-implemented diagnostic method of determining the genetic disease of a subject, the method comprising: acquiring assessed phenotypes of this subject, wherein the assessed phenotypes are selected from the group consisting of clinically assessed phenotypes, specific profiles in the transcriptome of the subject, specific profiles in the metabolome of the subject, changes in the transcriptome of the subject, changes in the metabolome of the subject; providing a database of genetic diseases linked at least in part to at least one relevant region in the genome of the subject and at least in part to at least one phenotypic characteristic; analyzing the genome of the subject; identifying changes in the analyzed genome by comparing it to at least one reference genome; ranking a plurality of possible genetic diseases of this subject related to the assessed phenotypes of this subject, characterized in that the ranking comprises comprising: assigning weight scores to each of the identified changes based on predetermined criteria, wherein assigning weight score to each of the identified changes based on predetermined criteria comprises a step selected from the group consisting of: assigning a score based on the presence or absence of an identified change in a gene in the analyzed genome of the subject; assigning a score to an identified change based on whether this identified change is known to cause a genetic disease or not; assigning a score based on the frequency of the variant in the database (22) of genetic diseases; running the genomic data of multiple family members of the subject and assigning a score to the identified change based on whether it segregates with a disease present in the other family members or if occurs de novo in the subject; running the genomic data obtained from different tissues of the subject and assigning a score to the identified change based on whether it segregates with a disease present in at least one of the tissues; assigning a score to the identified change by taking into account the strength of the association of an identified variant with a studied trait by using a statistical method to analyze the cosegregation of the identified change with a disease at a population and/or a family level, the statisitical method being selected from the group consisting of: a Bayesian analysis, an LOD score analysis, a TDT analysis and any combination thereof; assigning a score based on the type of identified change; assigning a score based on whether or not the identified change is present in a gene which is part of a cellular pathway already known to be involved in the pathophysiology of the assessed phenotype; assigning a score based on the evolutionary conservation of the identified change; assigning a score based on the pattern of inheritance within the family history of the subject; and any combination of the foregoing; searching the database (22) based on the assessed phenotypes thereby providing a first ranking of possible genetic diseases, each genetic disease being related to at least one genetic change indicative of that disease; adjusting the first ranking of possible genetic diseases based on the assigned weight scores; and providing a second ranking of possible genetic diseases based on the adjustment thereby providing for determining the genetic disease of the subject based on the second ranking.

In an embodiment, the step of identifying is performed without providing the user access to the identified changes.

In an embodiment, the method further comprises encrypting the analyzed genome prior to the step of identifying.

In an embodiment, the subject is a human subject.

In an embodiment, the genomic data of multiple family members is encrypted.

In an embodiment, the region is selected from the group consisting of a coding region, a gene, a non-coding region.

In an embodiment, the step of analyzing comprises sequencing.

In an embodiment, the change is selected from the group consisting of a mutation, a gene variant, an epigenetic change.

In an embodiment, the step of searching the database comprises searching by phenotypic characteristics or by keywords related to the assessed phenotypes.

In an embodiment, the method further comprises testing the subject for the possible genetic diseases based on the second ranking to determine the genetic disease of the subject.

In accordance with an aspect of the present disclosure, there is provided a diagnostic system for determining the genetic diseases of a subject, the system comprising: a database of genetic diseases linked at least in part to at least one relevant region in the genome of the genome of the subject and at least in part to at least one phenotypic characteristic; a data storage medium (20) comprising the analyzed genome of the subject, wherein changes in the genome of the subject have been identified; and a processor (17) comprising a user interface (16) in communication with the database (22) and the a data storage medium (20), the processor (17) providing for identifying changes in the analyzed genome, ranking a plurality of possible genetic diseases of this subject related to acquired assessed phenotypes of this subject, wherein the assessed phenotypes are selected from the group consisting of clinically assessed phenotypes, specific profiles in the transcriptome of the subject, specific profiles in the metabolome of the subject, changes in the transcriptome of the subject, changes in the metabolome of the subject, characterized in that the ranking comprises: comprising assigning weight scores to each of the identified the change based on predetermined criteria, searching the database (22) based on the assessed phenotypes thereby providing a first ranking of possible genetic diseases, each genetic disease being related to at least one genetic change indicative of that disease and adjusting the first ranking of possible genetic diseases based on the assigned weight scores thereby providing a second ranking of possible genetic diseases based on the adjustment thereby providing for determining the genetic disease of the subject based on the second ranking, wherein assigning weight score to each of the identified changes based on predetermined criteria comprises a step selected from the group consisting of: assigning a score based on the presence or absence of an identified change in a gene in the analyzed genome of the subject; assigning a score to an identified change based on whether this identified change is known to cause a genetic disease or not; assigning a score based on the frequency of the variant in the database of genetic diseases; running the genomic data of multiple family members of the subject and assigning a score to the identified change based on whether it segregates with a disease present in the other family members or if occurs de novo in the subject; running the genomic data obtained from different tissues of the subject and assigning a score to the identified change based on whether it segregates with a disease present in at least one of the tissues; assigning a score to the identified change by taking into account the strength of the association of an identified variant with a studied trait by using a statistical method to analyze the cosegregation of the identified change with a disease at a population and/or a family level, the statistical method being selected from the group consisting of: a Bayesian analysis, an LOD score analysis, a TDT analysis and any combination thereof; assigning a score based on the type of identified change; assigning a score based on whether or not the identified change is present in a gene which is part of a cellular pathway already known to be involved in the pathophysiology of the assessed phenotype; assigning a score based on the evolutionary conservation of the identified change; assigning a score based on the pattern of inheritance within the family history of the subject; and any combination of the foregoing.

In an embodiment, the data storage medium is selected from the group consisting of a CD, a DVD, a memory key, a chip, and a cloud.

In an embodiment, the communication between the processor (17) and the database is selected from the group consisting of a local communication and a remote communication.

In an embodiment, the degree of the similarity of genetic changes is based on a predetermined set of criteria.

In an embodiment, the genetic change comprises a gene variant In an embodiment, the genetic variant comprises a mutation. In an embodiment, the genetic variant comprises mutations in different genes pre-classified to belong to the same pathway. In an embodiment, the degree of the similarity of genetic changes comprises a mutation in the genome of the target subject which is identical to a mutation in the genome of at least one of the other subjects. In an embodiment, the degree of the similarity of genetic changes comprises a mutation in the same gene of both the target subject and at least one of the other subjects. In an embodiment, the genetic change comprises an epigenetic change.

In an embodiment, searching the database by comparing the assessed phenotypes of the target subject against the phenotypic characteristics of the other subjects and searching the database by comparing the changes in the encrypted analyzed genome of the target subject against the encrypted genomes of the other subjects are concurrently performed.

In an embodiment, matching the target subject with one or more of the other subjects based on the phenotypic similarity therebetween and matching the target subject with one or more of the other subjects based on the genetic variant similarity therebetween are concurrently performed.

In an embodiment, the genetic conditions comprise genetic traits. In an embodiment, genetic conditions comprise genetic diseases.

In an embodiment, the region comprises a gene. The initial ranking of all diseases in the database is based on the encrypted genomic information (i.e. the patient's different types of mutations in the gene corresponding to each disease) and the adjusted ranking on the phenotypic features. The presence of a variant will thus include the respective disease in the initial ranking in accordance with the weight assigned to the class that the variant belongs to. Examples of different classes of mutations used include (1) known pathogenic mutations, (2) nonsense and frameshift mutations leading to a premature stop codon, (3) missense mutations, (4) variants known to be benign). Diseases in which no variant was identified in the corresponding gene are filtered out prior to the second ranking. Similarly, diseases for which the patient's genome contains pathogenic mutations but do not meet a pre-set threshold of phenotypic similarity with the subject's assessed phenotype are also filtered out. An option of showing the final re-ranked list of diseases re-grouped based on the type of mutation identified in the corresponding gene is possible. Each sub-group contains diseases ranked in order of phenotypic similarity to the subject evaluated but the genes corresponding to all the different diseases within that group would contain the same type of mutation (e.g. known pathogenic mutations).

In an embodiment, sequencing, includes in-vivo sequencing.

In an embodiment, phenotype search is achieved via a search engine which gives the user different options matching the term entered or being typed (e.g. when one enters "macro" may be given the options "macrosomia", "macrocephaly") or adjectives qualifying it (e.g. when one searches "macrocephaly" may be given the option "progressive macrocephaly"), similar to HPO database discussed further below.

In an embodiment, phenotypic characteristics comprise chronicity of the feature (e.g. age of onset of that sign/symptom).

In an embodiment, the database containing the phenotypic features for each disease could list a different score for a given feature in relationship to a specific disease based on knowledge about the positive and negative predictive value of that sign/symptom in that disease. Similarly, negative scores can be given for terms entered in the search that remain unmatched compared to the features of a given disease, especially in when the corresponding feature has a high negative-predictive value for that disease. The frequencies listed for the features under some diseases in the HPO database are useful to this end.

In an embodiment, the database is provided locally and the processor is linked to this server. In an embodiment, a plurality of servers and/or a plurality of databases are provided. In an embodiment, the database is provided by a server and the processor is remotely linked to this server. In an embodiment, a plurality of servers and/or a plurality of databases are provided.

In an embodiment, the pre-set criteria for the overall impact score calculated for each genetic variant identified evolve as new knowledge is acquired and the impact score corresponds to the level of certainty for the variant's pathogenicity.

In an embodiment, the physician has the option to run in parallel the encrypted genomic data of multiple family members simultaneously to allow for comparisons to be taken into consideration when calculating the impact of a variant. By indicating each encrypted genome included in the search as "affected" or "unaffected" a different impact factor can be assigned to the variant based on whether it segregates with disease or if occurs de novo (i.e. variant not present in the parents of the patient).

In an embodiment, the physician has the option to run in parallel the encrypted genomic data obtained from different tissues of a patient simultaneously to allow for comparisons to be taken into consideration when calculating the impact of a variant. By indicating each encrypted genome included in the search as "affected tissue" or "unaffected tissue" a different impact factor can be assigned to the variant based on whether it segregates with disease such as cancer.

In an embodiment, weight assignment can take into consideration the strength of the associations of identified variants with studied traits. Different statistical methods analyzing the cosegregation of the identified variant with disease and/or integrating the predictions of different computational tools about a variant's pathogenicity can be used in the calculation of an "overall impact-score".

In an embodiment, statistical methods comprise Bayesian analysis to factor in results of different computational tools in the overall score, relative risk analysis, LOD score analysis, and/or Transmission Disequilibrium Test (TDT) analysis for each variant at a population and/or family level.

In an embodiment, an option to allow for the physician to adjust the default parameters of weight assignment is available through an interactive checklist. The physician can opt to assign a different contribution for a specific parameter (e.g. a much increased contribution for homozygosity when dealing with consanguinity) in the calculation of the variant's overall score.

In an embodiment, after viewing the adjusted ranking of possible genetic diseases, the physician has the option to discuss with the patient (or the family of the patient as appropriate) the physician's suspicion of a specific diagnosis. At this point informed-consent can be obtained and the patient can authorize the decryption of the relevant regions of the patient's genome so that the physician can perform a more careful analysis of the changes identified in the respective loci. This allows the physician to reassess the evidence supporting that a given diagnosis needs to be further pursued with clinical testing.

In an embodiment, along with the second ranking of possible genetic traits, the change in ranking for each genetic trait is provided. In an embodiment, the option to rank results based on the magnitude of change in ranking is also available.

In an embodiment, genetic trait comprises physical examination findings such as "microcephaly" or "short stature". In an embodiment, genetic trait comprises changes at the level of transcription or metabolome. In an embodiment, genetic trait comprises response of an individual to a medication. In an embodiment, genetic trait comprises complications/side-effects of using a specific medication. In an embodiment, genetic trait comprises infections associated with external sources of nucleotide sequence (i.e. infectious agents containing their own nucleotide sequence) affecting the host organism.

In an embodiment, data storage and/or analysis can be ontology driven. In an embodiment, genetic variant comprises identical mutation. In an embodiment, genetic variant comprises mutations in the same gene. In an embodiment, genetic variant comprises mutations in different genes pre-classified to belong to the same pathway. In an embodiment, genetic variant comprises a pattern of genetic variants. For example, variants in a group of patients sharing phenotypic features which are simultaneously present in multiple identical genetic locations in all of these patients and may act synergistically.

In an embodiment, one can use a spectrum of pre-defined criteria assigned a different weight each. These criteria correspond to the level of similarity shared between the genetic variants of individuals where different types of genetic variants are assigned different weights. For example, a group of individuals all sharing the same nucleotide change is assigned a higher score than another group having changes in the same gene which are not identical at the nucleotide level. Similarly, a group of individuals all having genetic changes in the same pathway but not all at the same gene will be ranked even lower.

In an embodiment, the final score of a genetic variant also takes into consideration predetermined criteria corresponding to the certainty for its pathogenic nature. For example, a group of individuals sharing phenotypic features all having changes in the same gene which are not identical at the nucleotide level may be split into two groups with the ones sharing known pathogenic mutations in the respective gene ranking higher than the ones sharing not-known pathogenic variants.

In an embodiment, a minimum threshold score of shared similarity is used at the level of the phenotype. This means that patients in the database whose phenotypic characteristics are not matching those of the target-subject beyond a certain level are filtered out from the first ranking.

In an embodiment, a threshold of a minimum number of subjects is required for a cluster to be considered as a separate entity.

In an embodiment, subjects whose extent of similarity falls in pre-set ranges are determined to be part of the same cluster. This means that two patients whose level of similarity in relation to the target-subject is not identical but very similar are clustered together based on predetermined criteria.

In an embodiment, a maximum number of clusters to be shown in the list provided after the first or second ranking is set in advance.

In an embodiment, the order of the steps is reversed and the initial ranking is based on the extent of sharing a similar genetic variant whereas the phenotype is used to adjust this ranking.

In an embodiment, genetic variant comprises a pattern of genetic variants. For example, in some cases predisposition to a genetic condition occurs by variants simultaneously present in multiple different genetic locations which are interacting. In this example, a group of patients sharing enough phenotypic features and variants in multiple identical genetic locations is also ranked high in the adjusted list.

In an embodiment, the phenotypic information of a cluster made available to the user at the end of the search comprises the diagnoses of the respective subjects when listed in the database.

Using a database with well-protected confidential information of real unrelated patients instead of a database of diagnoses has a major advantage: there is no need for a priori hypothesis for the importance of specific phenotypic features identified or pre-classification of variants in a database based on predicted pathogenicity. Ultimately, all the subjects sharing adequate phenotypic characteristics who also share a genetic variant cluster together. Next, If the diagnoses of some of the patients in the cluster under consideration are already known, they are listed in their profiles available in the database and can thus be accessed at the end of the search when browsing through the characteristics of the different clusters. One can browse through the results section and find out which phenotypic characteristics and which genetic variant are shared by all subjects classified in the same cluster. As a consequence, this tool would be very important to use during follow-up visits of patients not yet diagnosed since many new diseases will be constantly identified and the database profiles updated.

An advantage of the approach described is that the information of the subjects in the database remains confidential at all times since their genomes are encrypted. This allows for a standardized centralized database of patients with both known and unknown diagnoses which aids in diagnosing patients with atypical presentations of known genetic conditions or of genetic conditions not-yet identified. It also allows for subgrouping of patients currently diagnosed as having the same heterogeneous complex disease into new diagnostic dimensions based on both phenotype and genotype, thus redefining disease entities.

The advantage of reversing the order and having the initial ranking based on the extent of subjects sharing a similar genetic variant and the phenotype used to adjust this ranking is obvious in cases where the phenotypic features defining the disease entity are not as obvious (e.g. genetic syndromes which can have variable presentations not yet identified). This concept allows for clustering of subjects based on genetic similarities using pre-defined criteria but also makes use of the phenotype in a refining rather than restricting fashion.

Other objects, advantages and features of the present disclosure will become more apparent upon reading of the following non-restrictive description of non-limiting illustrative embodiments thereof, given by way of example only.

### BRIEF DESCRIPTION OF THE DRAWINGS

In the appended drawings, where like reference numerals denote like elements throughout and in where:
Figure 1 is a schematic representation of the genetic trait search system in accordance with an illustrative embodiment of the present disclosure;
Figure 2 is a flow diagram of an illustrative example of genetic trait search procedure executed by the genetic trait search system of FIG. 1 using the genetic conditions database 22; and
Figure 3 is a flow diagram of an illustrative example of genetic trait search procedure executed by the genetic trait search system of FIG. 1 using the subjects database 26.

### DETAILED DESCRIPTION OF ILLUSTRATIVE EMBODIMENTS

The present system and method allows for searching regularly updated databases which include phenotypically and genetically well characterized diseases with specific keywords which form the basis for ranking the results as is known in the art, yet with the addition of taking into consideration the patient's mutations identified by genome/exome sequencing when ranking the results. The system and associated method use an impact factor that corresponds to the level of certainty for the pathogenic (disease-causing) role of the mutation thereby appropriately adjusting the ranking of the search results based on this impact or weight factor without the physician having knowledge of the exact mutations identified by genome/exome sequencing.

In an embodiment, the genome/exome sequencing data of a patient is encrypted. In an embodiment, there is provided a method of analyzing this encrypted data in tandem with searching a database. Different mutations are assigned a different weight based on pre-set criteria which are integrated in the search application algorithm. Such criteria are typically used in the clinical evaluation of any identified variant that is found after genetic testing. The ranking of any given diagnosis is adjusted based on whether a pertinent genetic change was found in the target subject by genome/exome sequencing at the area of the genome (coding or non-coding region) known to cause or predispose to the respective/corresponding genetic disease.

In practice, the clinician does not need to look at the genome/exome sequencing results. In an embodiment the clinician does not have access to these results as they remain encrypted providing only the patient with full access to this confidential data. The foregoing provides several advantages; for example, consent is simplified as the patient does not need to discuss with the clinician which portions of the genome are to be sequenced. Consent will be obtained to explore the genetic causes explaining the phenotype/disease for which the patient is consulting the physician. This also simplifies counseling patients after results are obtained since counseling is focused on the disease at question. This way privacy and the principles of patient autonomy, non-maleficence, and informed consent are respected. Nevertheless, in special cases in which the clinician would need to have access to the encrypted data and the specific mutations therein a more elaborate counseling session with the proper consent is also provided by the present disclosure.

In one embodiment, the sequencing results are stored on a data storage medium, which may be, for example, a flash memory, USB key, CD, DVD and the like or any combination thereof, accessed either locally or remotely, for example on a cloud configuration. Furthermore, access to the data storage medium may be password protected.

The clinician uses this data storage medium to temporarily and anonymously upload the encrypted data to the search engine. In an embodiment, a BLAST (Basic Local Alignment Search Tool) or like tool search is performed. In bioinformatics, a BLAST is an algorithm for comparing primary biological sequence information, such as the amino-acid sequences of different proteins or the nucleotides of DNA sequences. A BLAST search enables a researcher to compare a query sequence with a library or database of sequences, and identify library sequences that resemble the query sequence above a certain threshold. Typically, the clinician enters a DNA sequence in a query box and the closest matched sequences appear in order of percentage of similarity. Similar available tools, such as BWA (http://bio-bwa.sourceforge.net/), which are used in the analysis of exome sequencing data are useful.

The patient is provided with the option of allowing the clinician to store the data for future clinical research or reference or to destroy the data once used.

In an embodiment, the present disclosure provides for genome/exome sequencing to be used as a screening test and not necessarily as a clinical diagnostic test. With this approach a clinical genetic test is still used to confirm or rule out the disease which the physician suspects as most likely. A non-limiting object of this method is not to rule out a disease by using genome/exome sequencing but rather to increase the diagnostic yield of a clinical evaluation in a more cost-effective fashion and to decrease the time to diagnosis. This method provides a better predictive value for known syndromes as compared to conventional approach and increases diagnostic yield. For the remaining unresolved cases, the clinician needs to resort back to conventional approaches thereby reducing false negatives as long as qualified medical geneticists perform the evaluation, at least in the early stages of medical genomics, until other health professionals are adequately educated regarding its value, limitations, and appropriate clinical use.

Conventional approaches often require multiple clinical visits and multiple genetic tests before a molecular diagnosis is reached. Each clinical genetic test is often very expensive. The present disclosure, which follows the existing "phenotype-first" medical model, provides for saving resources by better prioritizing the genes to be tested in a clinical lab. As the cost of genome/exome sequencing is exponentially decreasing, the present method is overall efficient from a resource, time and personnel point of view both in the clinic and the laboratory. Nevertheless, the clinician remains responsible for identifying, and clinically prioritizing testing for, the clinically suspected syndromes by using current clinical testing modalities to confirm a suspected diagnosis even if it was not the first hit in the search list.

The present disclosure simply provides an extra tool at the clinician's disposal to make the foregoing assessment thus reducing cost and time. Its limitations are minimized as long as qualified personnel performs the evaluation since for the remaining unresolved cases one needs to resort back to conventional approaches. This reduces false negatives, as well as, accounts for other known genetic explanations (e.g. epigenetic changes) not accounted for by exome/genome sequencing.

Given that the clinician does not have access to all the mutations of the patient under evaluation, obtaining informed consent is simplified and remains feasible after ordinary counselling. The patient only needs to consent that the clinician can explore the genetic causes which can potentially explain the specific phenotype/medical-issue at hand. This approach also simplifies counselling patients after results are obtained since counselling is focused on the disease in question, which is identical to current practice. No unwanted or unrelated results will become evident. The clinical terms entered in the search are selected based on the diseases of interest. Increased predisposition to cancer could still be identified when evaluating a child for a different presentation (e.g. cognitive deficits and skeletal findings) but this is only in the context of the clinical evaluation (e.g. NF1 mutations in the case of the "developmental delay AND skeletal findings" leading to Neurofibromatosis which is associated with specific well-documented malignancies). Preventing or optimizing management for such complications associated with a given genetic diagnosis is already part of the geneticist's responsibility.

Of course, in the event that the clinician is mandated to actively analyze the raw data, a special counseling session can be conducted to discuss all the potential challenges and findings. If a physician wants to actively analyze the raw data, a session with specialized counseling ensuring informed consent is provided.

With reference to the appended Figures, non-restrictive illustrative embodiments will be herein described so as to further exemplify the disclosure only and by no means limit the scope thereof.

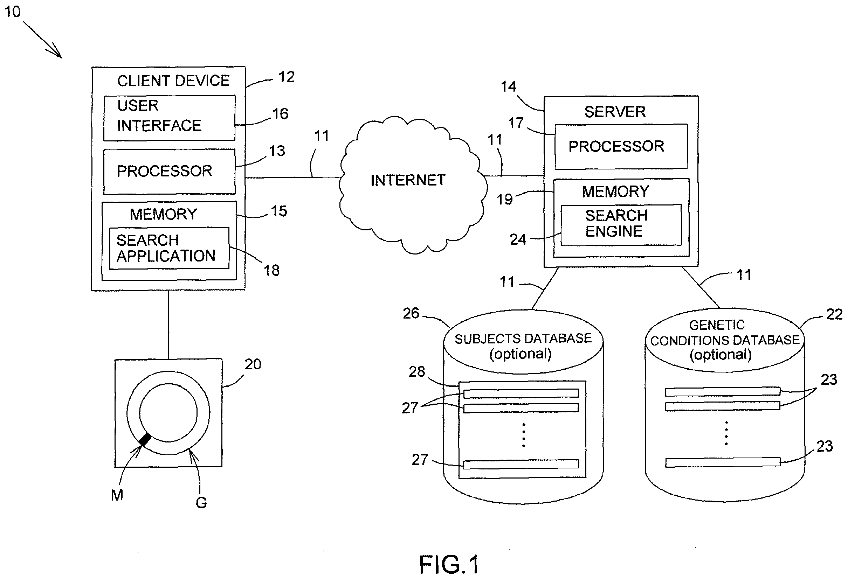

Referring to Figure 1, the genetic trait search system 10 generally comprises one or more client device 12, a server 14, a data storage medium 20 and either a genetic conditions database 22 or a subjects database 26, which are interconnected via various connections 11 (locally or remotely) such as, for example, Ethernet (broadband, high-speed), wireless WiFi, cable Internet, satellite connection, LAN, WAN, serial connection, cellular or satellite network, etc.

The client device 12 includes a user interface 16, for example a display and keyboard, a processor 13 and an associated memory 15 having stored therein a search application

The server 14 includes a processor 17 and an associated memory 19 having stored therein a search engine 24.

The data storage medium 20 in the illustrative embodiment is in the form of a DVD or a cloud configuration to give but two non-limiting examples, which includes the previously sequenced genome/exome G of a patient in an encrypted format and is accessed by the processor 13 executing the search application 18.

The genetic conditions database 22 is configured to store therein data 23 including known genetic conditions such as diseases with a genetic component (such as genetic syndromes) and associated relevant phenotypic characteristics and genetic regions/areas (loci) in the genome such as for example genes. Optionally, the database 22 can include under each gene listed the known variants of that gene. Optionally these variants can be classified in different categories e.g. known pathogenic (i.e. known disease causing) versus not-known pathogenic to give but two non-limiting example.

The subjects database 26 is configured to store therein, for a number of subjects 28, data 27 including one or more phenotypic characteristics for each subject 28 and the encrypted genome for each subject 28, wherein genetic changes in at least one relevant region in the encrypted genome of each subject 28 are indicative of a possible genetic condition or conditions.

In some cases, the subjects database 26 is configured to store therein, for a number of subjects 28, data 27 including the encrypted genome of each subject 28 and the assessed phenotypic characteristics of each subject 28 which are at least in part associated with genetic conditions and associated genetic loci in the genome of the given subject 28.

The search engine 24 is executed by the processor 17 to provide for searches of either the genetic conditions database 22 or the subjects database 26, conducted by the user through user interface 16 using the search application 18 and information provided by the data storage medium 20.

It is to be understood that although throughout the disclosure reference is made to separate client device 12, server 14, data storage medium 20, genetic conditions database 22 and subjects database 26, these may be implemented on one or more physical devices and may also be combined. It is to be further understood that the databases 22 and 26 may equally be implemented by a data structure within a computer memory.

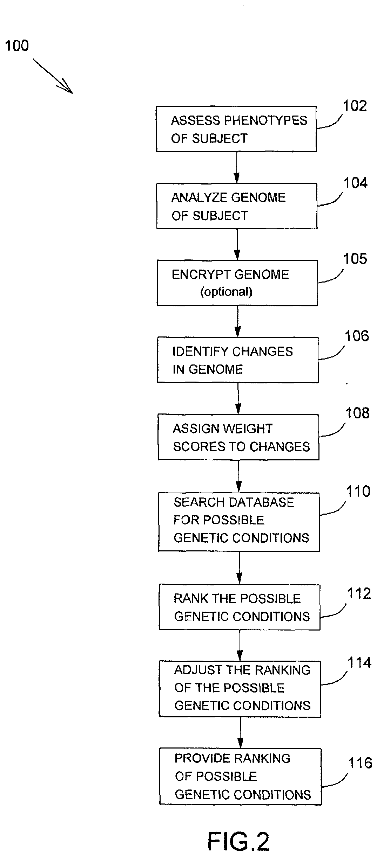

Referring to Figure 2, there is shown a flow diagram of an illustrative example of the genetic trait search procedure 100 used by the genetic trait search system 10 using the genetic conditions database 22. The steps of the procedure 100 are indicated by blocks 102 to 116.

The procedure 100 starts at block 102 where the phenotypes of a subject are assessed.

At block 104, the genome of the subject, for example from the data storage medium 20, is analyzed and optionally, at block 105, the genome of the subject is encrypted in order to ensure privacy.

At Block 106, changes in the (optionally encrypted) analyzed genome are identified by comparing it to a reference genome.

The clinician has access to the genomic data as part of the analysis but not the changes which are automatically identified by being compared to a reference genome without providing the user access thereto. I

At block 108, weight scores are assigned to each genomic change identified at block 106, the weight scores being based on predetermined criteria.

Then, at block 110, the genetic conditions database 22 is searched, via the search engine 24, using the assessed phenotypes of the subject from block 102, in order to identify possible genetic conditions, each genetic condition being related to at least one genomic change, identified at block 106, indicative of that condition.

At block 112, the identified possible genetic conditions are ranked based on degree of phenotypic similarity, for example the number of common phenotypic features.

Then, at block 114, the ranking of the possible genetic conditions is adjusted using the weight scores, assigned at block 108, for each genomic change linked to the possible genetic condition.

Finally, at block 116, a ranking of possible genetic conditions is provided via the user interface 16.

It should be noted that blocks 104, 105, 106 and 108 may be performed prior to block 102. There may be cases, for example, where the genome of the patient has already been sequenced at birth prior to any detection of clinically significant phenotypes.

It should be further noted that in embodiment procedure 100 may be automated until after block 114 to ensure the privacy of the subject. Accordingly, the physician would have his access restricted to the genetic areas corresponding to the ones related to the genetic conditions provided by block 114. In practice, there may be some access to the genomic data before the block 114 in order to, for example, manipulate the data and "clean it". However, no access to the variants is provided.

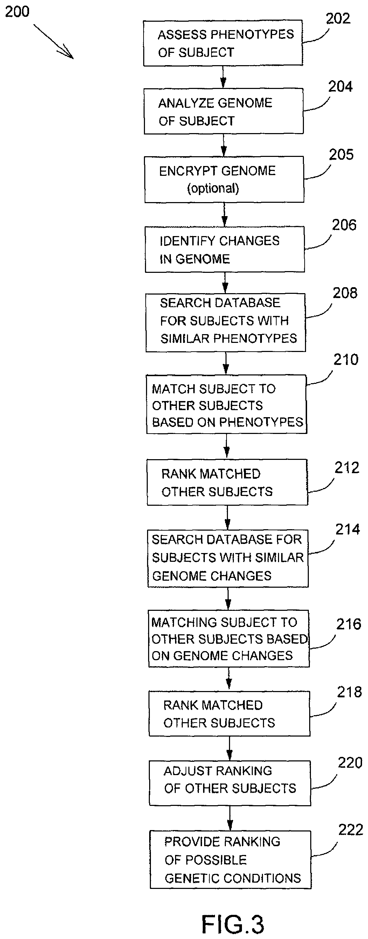

Referring to Figure 3, there is shown a flow diagram of an illustrative example of the genetic trait search procedure 200 used by the genetic trait search system 10 using the subjects database 26. The steps of the procedure 200 are indicated by blocks 202 to 222.

The procedure 200 starts at block 202 where the phenotypes of a subject are assessed.

At block 204, the genome of the subject, for example from the data storage medium 20, is analyzed and optionally, at block 205, the genome of the subject is encrypted in order to ensure privacy.

At Block 206, changes in the (optionally encrypted) analyzed genome are identified by comparing it to a reference genome.

At block 208, the subjects database 26 is searched, via the search engine 24, using the assessed phenotypes of the subject from block 202, in order to identify other subjects with phenotypic similarities.

Then, at block 210, the subject is matched to other subjects of the subjects database 26 based on their phenotypic similarities and, at block 212, the matched other subjects are ranked based on the degree of phenotypic similarities, for example the number of common phenotypic features.

At block 214, the subjects database 26 is searched, via the search engine 24, using the genomic changes, identified at block 206, in order to identify, amongst the other subjects sharing common phenotypic similarities to the target subject, other subjects who additionally share one or more similar genomic changes with the subject. A predetermined phenotypic similarities threshold may be used to determine amongst which other subjects the genomic search will be executed.

At block 216, the subject is matched to other subjects of the subjects database 26 based on their similarities in genomic changes using predefined criteria and, at block 218, the matched other subjects are ranked based on their genomic changes similarities. In an embodiment, predefined criteria are used, for example a group of individuals with similar phenotypic features sharing an identical mutation is assigned a higher score than if the same group had different mutations present in the same gene.

Then, at block 220, the ranking of the other subjects is adjusted using the genomic changes ranking of block 218.

Finally, at block 222, a ranking of possible genetic conditions is provided via the user interface 16 based on the ranking of the other subjects provided at block 220, the ranking being indicative of the possibility that the subject shares the same genetic conditions with a corresponding other subject.

It should be noted that blocks 204, 205, 206 and 208 may be performed prior to block 202. There may be cases, for example, where the genome of the patient has already been sequenced at birth prior to any detection of phenotypes.

It is to be understood that in an embodiment genetic trait search procedures 100 and 200 may be combined in order to provide a ranking of possible genetic conditions based on data from both the genetic conditions database 22 and the subjects database 26.

In an embodiment, the genetic conditions database 22 include all single gene mendelian disorders and complex traits for which well-characterized and validated genetic variation results in significant contribution to the phenotype. Access to both the phenotypic characteristics for each genetic disease and all the genetic changes shown, to date, to cause or predispose to this genetic disease are available.

In an embodiment, both the phenotypic characteristics and the genetic variants known for each genetic condition are located in a single standardized genetic conditions database 22.

The present method provides for learning more about new phenotype-molecular correlations through properly designed, consented and approved research projects. Discovery of new genes for known syndromes, discovery of new syndromes or refinement of the phenotype of a known syndrome is facilitated. For example, in conventional approaches only patients who fit very closely all or almost all characteristics of a described genetic syndrome are tested for the genes involved. The present disclosure, on top of enabling earlier diagnosis via clinical utilization of genomic technologies [e.g. Jimenez-Escrig et al. Muscle Nerve. 2012 Apr;45(4):605-10. PMID: 22431096], provides for the partial matches of patients to be identified and their characteristics enrich and refine the spectrum of clinical characteristics of any given syndrome.

The genetic conditions database 22 can be updated regularly as such whatever genetic change will be confirmed to cause or predispose to a disease will be added and as such, one is able to search the regularly updated genetic conditions database 22, which includes all phenotypically and genetically well characterized diseases, based on both genotypic and phenotypic characteristics of the specific patient being evaluated. When building the genetic conditions database 22 all "well documented phenotypes", "genetic traits", and "genetic diseases", including more common complex diseases can be included as long as they already have a well documented genetic change with a specific impact correlating to a specific weight factor thereby affecting their ranking. For example, BRCA1 mutations predisposing to a more complex disease, namely, breast cancer. Moreover, mutations in genes or regulatory elements not yet identified to play a role in a genetic disease but suspected to do so based on knowledge of them being linked to a pathway already documented to be involved in a specific disease (e.g. Ras pathway for SLO syndrome) may be assigned a specific, albeit lower, weight. As the database garners more information about modifier alleles (different genetic mutations that interact to predispose a patient to a disease) or epigenetic changes these can also be integrated in the algorithm and be useful for more complex phenotypes. For example, genetic modifiers of cancer risk in Lynch syndrome have been reported: shorter IGF1-CA repeats are associated with an increased risk for colon cancer and earlier age of onset among individuals who have a mutation in an MMR gene.

Similarly to the genetic conditions database 22 with genetic diseases a database with different genetic traits such as specific sub-phenotypes (rather than the genetic disease as an entity) identified during physical exam (e.g. "microcephaly") can be generated. All genes/genetic loci known to lead to a phenotype comprising the respective sign will be listed. Several sources, including textbooks describing the genetic pathophysiology of such sub-phenotypes will be useful to this end (e.g. Epstein et al: Inborn Errors of Development. Oxford University Press; 2008).

Similarly, more complex sub-phenotypes such as biochemical profiles can be useful. For example, schizophrenia has high heritability but complex inheritance and is suspected to constitute a constellation of different pathogenetic processes leading to a similar phenotype. Some of these pathogenetic processes may ultimately have a specific signature not only at the level of the genome, but also at the level of the epigenome, transcriptome or metabolome. If the known such signature profiles are listed in the genetic conditions database 22 used by the search engine 24, the data generated at the time of the clinical evaluation from RNA sequencing and/or metabolomic profiling or even epigenomic changes over time in such a (symptomatic) patient could serve as a phenotypic trait refining the search. For example, it is already known that in some biochemical genetic disorders, a.k.a. inborn errors of metabolism (IEM), psychiatric manifestations such as psychosis may be a predominant feature or the only presenting sign for many years leading to misdiagnosis for diagnoses such as schizophrenia. Several IEM are treatable and delay in initiating treatment can lead to permanent neurological damage or even early death. Storage diseases involving multiple small organelles (lysosomes, peroxisomes, mitochondria), iron or copper accumulation, as well as defects in other pathways (e.g. defects leading to hyperammonemia or homocystinemia and cobalamin deficiency) can present as psychosis. Pathogenic mutations in the respective genes and biochemical profiles, identified via metabolomics, characteristic of such a disease and indicative of the respective imbalance can serve to adjust the ranking of the genetic disease associated with psychosis and help the physician suspect and subsequently diagnose the respective rare IEM, which at early stages could be presenting as schizophrenia.

### Subjects database

Similarly to the genetic conditions database 22 with genetic diseases, a database containing information about the phenotypes and encrypted genomes of consenting human subjects can be generated. In subjects whose diagnoses have been confirmed their profile will also contain this information. The subjects database 26 can also include the encrypted genomes of consenting healthy subjects and their phenotypic features (including metabolomic, transcriptomic profiles, asymptomatic status) which can be updated at different points of time. The entry for each consenting subject will be password protected and the password only available to the patient. Each entry is coded with a specific number and the patient's identity remains protected at all times. This subjects database 26 is managed similarly to the guidelines for "biobanks". Specific protocols, ethical approval and special counseling for informed consent of the participating individuals are needed if someone wants to use the genome/exome data for research purposes. A subject from database 26 can opt to not be contacted for any research projects.

In an embodiment, only certified physicians can enter new data in the subjects database 26. The physician is given an option to indicate which phenotypic characteristics entered in the patient's profile are most striking/reliable. An option of assigning a weight about their confidence with regards to the presence or absence of each sign/symptom submitted is available. The option of a questionnaire by category (respiratory, cardiovascular etc.) aids in the standardization of phenotypic information entered for each new patient in the subjects database 26.

In one embodiment, only exome sequencing is performed. In another embodiment, sequencing will include non-coding regions to include non-coding genetic changes (e.g. mutation in regulatory elements) with a confirmed impact causing or predisposing to a specific phenotype. For example: "campomelic dysplasia" can be caused by SHOX enhancer deletion not involving the coding region of the SHOX gene itself. Many more such changes are expected to be found.

The genetic conditions database 22 is sequentially improved as more genetic defects become known. The genetic conditions database 22 includes genetic defects confirmed to cause or predispose to (alone or in combination with another factor) the genetic disease/trait.

The genetic conditions database 22 used can sort genetic loci under specific phenotypes based on the evidence of involvement in each. As previously stated, the genetic traits/phenotypes under which different genes/loci involved are listed include known genetic diseases, or specific sub-phenotypes like "microcephaly", or even more complex phenotypes such as biochemical profiles identified by metabolomics.

The data from a patient's genome sequencing can be used for different medical problems presenting at different times of a person's lifetime. The design of the presented approach could render obtaining genomic data during childhood or even newborn period practical, ethical and clinically useful. This is true because the genomic data can remain encrypted and thus only indirectly be useful for medical problems presenting at different points of a person's lifetime.

### Weight/impact factors

The predetermined criteria integrated in the algorithm of the search application 18 correspond to the level of certainty for the pathogenicity of each genetic variant identified and evolves as new knowledge is acquired. They are equivalent, at any time, to the criteria used in the clinical evaluation of variants identified after a clinical genetic test. Some genetic variants can be unequivocally interpreted as pathogenic or normal variants on the basis of extensive clinical experience. In other cases, clinical experience is insufficient and pathogenicity is inferred based on extensive computational analysis.

Each variant identified can be classified to a class of variants defined based on existing published criteria (e.g. http://cmqsweb.shared.hosting.zen.co.uk/BPGs/Best_practice_Guidelines.htm or Richards et al. Genet Med 2008, 10(4):294-300). These guidelines for variant interpretation can be used to classify each variant identified in one of four or six different groups with a different "impact factor" potentially assigned to each group
Alternatively, an overall score will be automatically calculated for each variant identified by simultaneously taking into consideration different factors each contributing a specific weight to the overall score. It is the overall score assigned to the variant which is used to adjust in each patient the ranking of the initial phenotype-based ranking of possible diagnoses.

Examples of such factors include: (1) Number of sequencing reads covering the position which is indicative of the certainty that the variant is real and not an artifact; (2) Frequency of the variant in genomic variation databases; (3) Type of mutation (silent, splicing, missense and effect on aminoacid properties, nonsense, frameshift, intronic, mutation involving regulatory elements etc.); (4) Whether the identified variant has been previously been reported in disease in general and in the specific phenotype of interest more specifically; (5) Functional data, presence of in-vitro evidence supporting the pathogenicity of the variant; (6) Whether the variant is present in a gene which is part of a cellular pathway already known to be involved in the pathophysiology of the phenotype of interest; (7) Evolutionary conservation (conservation across species of the position where the variant is found).

Information about the pattern of inheritance and family history can also be factored in the weight assignment process and thus have an impact on the overall score calculated for the variant identified. For example: (1) When suspecting Autosomal Recessive pattern of inheritance, assigning a different impact for a variant present in homozygous state can be done. Having a different weight for homozygosity versus compound heterozygosity state of the variant analyzed can be important especially in the context of consanguinity; (2) When suspecting X-linked (recessive/dominant) pattern of inheritance the weight of variants present on the X-chromosome can be adjusted accordingly; (3) Presence or absence of the variant in other affected/unaffected family members by simultaneous comparison of the encrypted genomes of consenting family members; (4) Presence or absence of the variant in different tissues (affected versus unaffected) obtained from the affected individual. Simultaneous comparison of the encrypted genomes derived from the affected and unaffected tissues to assess whether the variant segregates with disease can be helpful for impact factor assignment in cases where mosaicism is present (e.g. de novo mutations in cancer).

Different statistical methods can be useful in the calculation of the overall impact score: e.g. relative risk, LOD score analysis, and/or Transmission Disequilibrium Test (TDT) analysis for each variant at a population and/or family level. Similarly, Bayesian analysis can be used to factor in the results of different computational tools in the overall score.

An option to allow for adjusting the default parameters is possible through an interactive checklist. One can adjust the weight/contribution of a specific parameter in the overall calculation of the variant's score. Alternatively, one can opt to include more or less factors than the selected default parameters in the calculation of the overall variant score.

The phenotype score is a function of how closely the subject's phenotypic features used in the search match with those of the known phenotype of the disease.

To minimize the risk for incidental findings a cut-off score for phenotype contribution can be set and the search application 18 algorithm can be trained to ensure that the phenotype drives (has the highest impact on) the ranking.

To minimize the risk for incidental findings the weight assigned to the phenotype contribution can be set to be much higher than that of the genetic variants so that the clinical features remain the principal driving force of the search.

Weight factors and the assigning process can be modified based on new knowledge. For instance, they can account for established gene-gene or gene-environment interactions in genetic diseases (e.g. Lincoln et al. 2009: Epistasis among HLA-DRB1, HLA-DQA1, and HLA-DQB1 loci determines multiple sclerosis susceptibility).

The data generated at the time of the clinical evaluation from RNA sequencing and/or metabolomic profiling or even epigenomic changes over time in a specific patient could serve in the calculation of the overall impact score for a variant. For example, in a given patient, the presence of characteristic changes at the level of the epigenome, transcriptome or metabolome known (or suspected based on other evidence as described below) to be associated with a specific genomic variant present in that patient, will be automatically identified and consequently raise further the overall impact score of that genomic variant in this specific patient. The (degree of) biochemical imbalance will constitute an important factor in predicting the pathogenicity, and thus in calculating the variant's "impact factor". To facilitate this, the databases used by the search engine 24 will list the different expression, metabolomic etc. profile signatures associated with each variant.

Candidate genes/loci for each genetic trait can also be included in the genetic conditions database 22, albeit with a lower "impact-score", representing the amount of evidence/probability of the genetic locus' involvement in the respective trait. These would be pre-selected candidate genes/loci, including regulatory elements, not previously documented to i.e. already known to be associated with a genetic trait but known to play a role in a pathway already known to be associated with a genetic trait. Pre-selected candidate genes/loci could represent loci with epigenomic, transcriptomic, metabolomic, or proteomic (e.g. protein interactome) evidence supporting their role in such a network, which when defected is known to lead to the genetic trait at hand. For any given patient, when the ranking of a specific genetic trait is changed significantly after taking the patient's genome into consideration, first all the genes documented to be associated with the genetic trait will be explored. If no mutations are found in these genes, consent to explore the *pre-selected* candidate genes/loci for the genetic trait at hand for mutations/changes can be obtained. This can lead to the identification of novel genes associated with known genetic traits/diseases.

Simplified non-limiting examples for assigned "mutation impact weights" are presented below. For the sake of simplicity, in each of the cases below the focus is placed on one parameter as if it was the only parameter contributing to the overall score assigned to a variant.
(a) All known common population variants based on the maintained up-to-date variants-database are identified as such and are assigned no or minimal weight.
(b) If the genome sequencing of the patient yields a mutation previously reported to cause (or predispose to) a known syndrome, the score of that syndrome would be multiplied by a multiplication factor (impact factor or weight) such as a number X wherein X=5, for example, so it would come higher on the resulting list.
(c) If a novel mutation is found in a gene known to be linked with a specific syndrome the multiplication factor (weight) could be 4 (X=4) in the case of a severe mutation like an early stop-codon versus weight of 3 (X=3) if a novel missense mutation.
(d) If genome sequencing of the patient yields a mutation in a gene not previously reported to cause this disease but involved in the pathway already known to contain genes leading to the syndrome (e.g. Ras pathway for SLO syndrome) a lower weight could be assigned such as X = 3 if this mutation is a severe mutation/early stop codon, or X = 2 if this mutation is missense mutation changing the property of the amino acid.
(e) Information about family history can be integrated in the algorithm assigning the weight/impact factor of the different mutations. For instance, when evaluating a consanguineous couple (or another case where autosomal recessive inheritance is suspected), the clinician can have the option to repeat the search or add a final step, adding more weight (e.g. X = 6) for mutations present in both copies of any given gene. In the case of a patient from a consanguineous family, an even greater weight (e.g. X=7) could be assigned to identical mutations present in both copies of a specific gene. Similarly, if x-linked transmission is suspected, the database could be searched again and an increased weight could be assigned to mutations on the x-chromosome.

In another embodiment, instead of using multiplication factor, a different mathematical function/relationship can be used for the score adjustment. The numbers (X) discussed above can be replaced by "unknown variables" V,Y,Z,T, A where V>Y>Z>T>A and the ratio of V/Y, or Y/Z, or Z/T, or T/A may not be equal to one. The variables considered can be limited to two ("known pathogenic" versus "NOT"; with a different score assigned to each category) or encompass different weights based on the degree of certainty for the pathogenicity of a variant.

In an embodiment, a disease present in the first ranking can be adjusted upward or downward on the second ranking. For example, the absence of a genetic variant in the genes corresponding to a specific disease can be used to decrease the overall score of that entry in the second ranking. This is achieved by multiplying by zero or a fraction number the score of these diseases during the process determining the second ranking (where, for example, absence of any genetic variant can be assigned a zero; while presence of a variant predicted to be benign can be assigned a score of 0.1, 0.01 etc. ensuring that the respective diagnosis stays on the list but in a lower position).

In an embodiment, the weights assigned both in the case of genetic variants and phenotypic features can be based on training the software in a standardized environment (e.g. standardized database, nomenclature, phenotypic evaluation/terminology, methodology of data collection and database update) using real patients' data. This can help to adjust the score/weight assignment process. For example, it helps to refine the prior/baseline probability when using Bayesian analysis of the data. Moreover, the data of successfully diagnosed patients can sequentially aid in refining weight assignment of different phenotypic features in respect to a specific diagnosis based on their positive and negative predictive value with respect to a given diagnosis.

In an embodiment, ranking of possible genetic conditions of a subject related to the assessed phenotype and the encrypted genome of this subject can be achieved using the subjects database 26 containing information about the phenotypes and encrypted genomes of other human subjects. In this case, there is no need of a priori weight assignment to specific phenotypic features or genetic variants; it is the extent of similarity of the subject's phenotypic and genomic data with that of different patients in the database, as determined based on pre-defined or predetermined criteria, that ultimately ranks the different clusters each of which represents patients who may have the same genetic diagnosis.

More particularly, the phenotype of the patient is searched against the phenotypes of the patients in the subjects database 26. For example if the clinician searches for four phenotypic characteristics, certain subjects in the subjects database 26 will also have all four traits in their profile and will thus be ranked as the highest cluster. Other subjects will have three traits so they will be ranked second from top and so on. That is first ranking. For the second ranking, the encrypted genome of the patient is searched against the entire encrypted genomes of the subjects in the subjects database 26 who are sharing a predetermined threshold of phenotypic characteristics with the patient. As such, the subjects who match with the patient based on the initial phenotype search have their genomes concurrently compared with the patient in order to identify whether at least some of these subjects share a genetic variant among themselves which is also present in the target subject. Therefore, if the clinician finds a genetic change in a large number of subjects who share the same phenotypic characterisitcs as the patient in question (or target subject) and that change is also present in the genome of the target subject, there is a strong probability that this change causes or predisposes to the said phenotypic characteristics. The extent of this probability can be assessed using statistical methods but a simplified example will follow to better convey the concept. If the clinician finds a group of subjects that has all four phenotypic characteristics and a mutation somewhere in the genome (e.g. in Gene X) which is exactly the same in all and is present in the patient in question, this group will have their rank adjusted to the top of the adjusted list. Another group of subjects will have all four phenotypic characteristics and a mutation in Gene X but not exactly the same mutation as in the patient, as such this latter group will have their rank adjusted downwardly as the 2nd group in the list. Still another group of subjects will have all three phenotypic characteristics and the exact same mutation in Gene X as the patient and so on.

Regarding the pre-defined or predetermined criteria with respect to phenotypic or genomic similarity, the clinician can use a spectrum of pre-defined or predetermined criteria which are each assigned a different weight score. These criteria correspond to the level of similarity shared between the individuals where different types of genetic variants are assigned different weight scores. For example, a group of individuals all sharing the same nucleotide change is assigned a higher score than another group having changes in the same gene which are not identical at the nucleotide level. Similarly, a group of individuals all having genetic changes in the same pathway but not all at the same gene will be ranked even lower.

In an embodiment, the final weight score of a genetic variant also takes into consideration predetermined criteria corresponding to the certainty for its pathogenic nature. For example, a group of individuals all having changes in the same gene which are not identical at the nucleotide level may be split into two groups with the subjects sharing known pathogenic mutations in the respective gene ranking higher than the subjects sharing not-known pathogenic variants.

In an embodiment, a minimum threshold score of shared similarity is used at the level of the phenotype. This means that the other subjects in the subject database 26 whose phenotypic characteristics are not matching those of the target subject beyond a certain level are filtered out from the first ranking.

In an embodiment, a threshold of a minimum number of subjects is required for a cluster to be considered as a separate entity.

In an embodiment, subjects whose extent of similarity falls in pre-set ranges are determined to be part of the same cluster. This means that two subjects in the subjects database 26 whose level of similarity in relation to the target subject is not identical but very similar are clustered together based on the predetermined criteria.

In an embodiment, there is a predetermined maximum number of clusters that are to be listed after the first or second ranking.

In an embodiment, genetic variant comprises a pattern of genetic variants. For example, in some cases predisposition to a genetic condition occurs by variants simultaneously present in multiple different genetic locations which may be interacting to cause the genetic condition. In this example, a group of patients all sharing enough phenotypic features and variants in multiple identical genetic locations is also ranked high in the adjusted list..

In an embodiment, similarity at the level of the phenotype comprises similarity between transcriptomic or metabolomic profiles of the subject and at least some of the patients in the subjects database 26.

In an embodiment, similarity at the level of the phenotype comprises similarity in changes of transcriptomic or metabolomic profiles in the subject and at least some of the patients in the subjects database 26.

In an embodiment, similarity at the level of the genotype comprises different levels of match between the encrypted genome of the subject and those of the patients in the subjects database 26 each assigned a different score: e.g. different score when there is a match as (1) an identical mutation, (2) mutations in the same gene, (3) mutations in different genes pre-classified to belong to the same pathway.

In an embodiment, subjects whose extent of similarity falls in pre-set ranges are determined to be part of the same cluster.

In an embodiment, a maximum number of clusters to be shown in the list provided after the first ranking is set in advance.

In an embodiment, a maximum number of clusters to be shown in the list provided after the second ranking is set in advance.

In an embodiment, the order of the steps is reversed and the initial ranking is based on the extent of sharing a similar genetic variant whereas the phenotype is used to adjust this ranking.

In an embodiment, the first ranking is based on the extent of sharing a similar genetic variant and the phenotype is used to adjust this ranking. The advantage of this is apparent in cases where the phenotypic features defining the disease entity are not as obvious (e.g. genetic syndromes which can have variable presentations not yet identified). It allows for initial clustering of subjects based on genetic similarities using pre-defined criteria but also makes use of the phenotype albeit in a refining rather than restricting fashion.

### EXAMPLES

The present method will be further described by the following non-limiting examples.

A patient P referred to genetics for hypotonia suffers from Smith Lemli Opitz (SLO) syndrome. The patient P's exome has been sequenced. According to the crude data from exome sequencing, patient P has a mutation in the DHCR7 gene known to cause SLO. The patient is clinically evaluated by a medical geneticist following exome sequencing. The geneticist is not aware of the mutation in the DHCR7 gene nor that the patient had SLO syndrome. The only positive indications the geneticist identifies as important during the evaluation are "hypotonia" and a "heart defect". The geneticist performed two searches, a standard search as well as search in accordance with the present disclosure.

The standard search was performed on the OMIM (Online Mendelian Inheritance in Man) website, a database of human genes and genetic disorders. The search results for the keywords "hypotonia AND heart defect" garnered 175 hits. SLO was ranked #23 on this list.

The search in accordance with the present disclosure was also performed on the OMIM website in tandem in conjunction with the encrypted sequencing data. The geneticist did not look at the genome/exome sequencing data per se, the geneticist simply ran in tandem with the clinical criteria "hypotonia AND heart defect" the ranking system of the present method during the search which took into consideration all the mutations identified by genome/exome sequencing assigning different weights or impact factors depending on the type of mutation.

Given that the identified pathogenic mutation in DHCR7 was automatically assigned a high weight (e.g. X), this time the cumulative score of SLO (Y x X) was relatively higher than that of most other diseases on the list. Hence, this time, SLO was ranked higher on the search results list and the geneticist entertained the diagnosis of SLO. The physician suspected SLO and decided to send for DHCR7 gene clinical testing to confirm the diagnosis. In this example, as per current practice, the geneticist before undertaking clinical molecular testing could have chosen to send a less-expensive non-molecular lab test named a sterol profile which if positive would have further increased the physician's a priori suspicion for SLO. Similarly, the physician could have discussed his suspicion of SLO syndrome with the patient (or the family of the patient as appropriate) and obtained consent to specifically analyze, in collaboration with the clinical molecular lab director, the patient's encrypted exome sequencing data in DHCR7 gene before ultimately pursuing the clinical diagnostic test. This would have allowed for a more careful analysis of the changes identified by exome/genome sequencing in this locus and provided further evidence to support that the diagnosis of SLO syndrome needed to be entertained.

Another patient P2 presents to clinic with fever and respiratory symptoms/signs. The clinician aims to identify the infectious agent to treat the patient with the right medication as early as possible. A database with the nucleotide sequence of each bug known is available. The clinician collects a blood or sputum sample from the patient and sequences/analyzes the total DNA present in the sample. The clinician then searches the database for "respiratory infection" as the phenotypic characteristic. As a result of this search a list of the names of all pathogens known to cause a respiratory infection is provided (e.g. in alphabetical order or in order of geographic prevalence). The search engine is then automatically comparing the sample's total DNA to the reference human genome sequence and/or unaffected (not-infected) family members. The non-matching DNA, corresponding to the DNA of the infectious agent, is automatically identified and compared against the database containing the nucleotide sequences of all bugs known. The extent of similarity serves to determine the score/weight assignment for each pathogen and to subsequently adjust the ranking of the possible infectious causes. This enables the physician to identify the infectious cause and treat the infection as early as possible (e.g. as early as fever develops).

In this example, instead of providing the list of infectious agents one could arrange to automatically provide, while respecting the same order, the corresponding medication targeting each of these infectious agents. Moreover, if there are several medications which could effectively treat a specific infectious agent, the final ranking of these medications could also take into consideration the genomic information of the host. For instance, the presence of a genetic variant in the host which has been documented to modulate the response to a specific medication or increase the risk for specific side-effects can be automatically factored in the overall score/weight assignment of that medication. For this to occur, the database will need to include the medication to which each infectious agent responds and under each medication the variants known to play a role in its effectiveness or side-effects.

There exist already examples in the literature suggesting that next-generation sequencing will be soon used in routine clinical microbiology practice (Didelot et al. Nat Rev Genet. 2012 PMID: 22868263).

Example of pharmacogenomics: genetic variation plays a key role in adverse reaction to drugs as well as to differences in the effectiveness of drug treatments. A good example of using pharmacogenetics clinically is testing for CYP2C9 (MIM #601130) and VKORC1 (MIM #607473) variants conjointly to determine dose requirements and hence susceptibility to adverse drug reactions related to warfarin. Such applications would be particularly valuable for many elderly patients and others with chronic diseases who must take many medications concurrently (Tucker et al. Massively parallel sequencing: the next big thing in genetic medicine, Am. J. Hum. Genet. 85 (2009) 142-154).

A patient P3 presents to clinic with an adult onset neurological disease characterized by severe ataxia, dystonia, psychiatric symptoms and white mater changes on brain MRI. After extensive testing the diagnosis remains unknown since the genetic syndrome (GS) of the patient P3 has never been described before. However, several other patients in the centralized database containing the phenotypic and encrypted genomic information of a large number of patients also have the same not yet-identified diagnosis (GS). The cause in most of these patients is a mutation in the same gene (GSG). The patient P3 undergoes a lumbar puncture and a CSF sample is collected. The metabolome of the CSF sample is analyzed (i.e. all the different metabolites in the CSF of the patient are identified and analyzed). In the centralized database, some of the subjects also have CSF metabolomic data available in their profiles. These CSF data are already organized in the database in different clusters based on similarity of the profiles. These clusters can be depicted similar to current ontology/ phylogenetic tree analysis diagrams. When the patient P3's CSF metabolomic data is searched as a phenotypic feature against the existing centralized database he is classified by the software in the same cluster as patients having similar metabolomic profiles (first ranking). This ranking of different clusters is adjusted by the software to maximize the score assigned to the cluster containing all the patients in the database with the features of GS disease present in the patient P3 and entered in the search and a mutation in the same gene, in this example, GSG gene. By clicking on the top-ranked cluster in the results section of the search the physician is able to access its characteristics, including the phenotypic features and the "gene variant" shared by all patients in this cluster (here mutations in GSG gene). Hence, this system enabled the physician to identify the novel genetic syndrome in the patient P3, the genetic cause and metabolomic profile shared by all patients in the same cluster. After careful verification and validation of the findings in his patient the physician publishes this new syndrome and the data about the underlying pathophysiology (genetic, metabolomic data). He then updates his patient's profile to include the new syndrome as the confirmed diagnosis. When another patient with GS syndrome from the top-ranked cluster mentioned above presents in follow-up visit to her physician, the physician runs the software and takes advantage of the new diagnosis entered in the database.

In an embodiment, in the above example the metabolomic data of the patients in the database are not already organized in different clusters based on similarity of the profiles. Approaches such as Principal Component Analysis (PCA), Weighted PCA, and Vector Analysis are used to compare the metabolomic data of the target-subject with those of the subjects in the database and rank the different clusters accordingly.

In an embodiment, a pre-set level of statistical significance, e.g. a p-value can be used in these analyses.

In an embodiment, similar to the CSF metabolome being used as a phenotypic feature other sources of metabolomes (e.g. blood, urine, lipids) can be used.

In an embodiment, similar to the CSF metabolome being used as a phenotypic feature, clinical features can also be used. Principal Component Analysis (PCA) and Vector Analysis also apply in this search
Regarding the next example, it should be noted that as documented in the literature, psychosis can be the isolated presenting feature for many years in several inborn errors of metabolism (such as cobalamin C, Nieman Pick C, X-linked adrenoleukodystrophy, Metachromatic Leukodystrophy, and Wilson's disease) some of which are treatable, especially if diagnosed in early stages.

Three different patients (A, B, C) present in clinic with psychosis. All three patients have a different inborn error of metabolism (IEM) associated with psychosis (IEM1, IEM2, IEM3, respectively; where IEM1 is caused by changes in "gene 1"; IEM2 in "gene 2"; IEM3 in "gene 3"). However, because their metabolic diseases are in their very early stages and no other symptoms are present, nor neurological or other signs were found during the physical examination. The physician examining these three patients was not aware of their underlying diseases. The physician's evaluation is only positive for psychosis.

A "Sample database" is provided containing 15 well-known IEMs (including IEM1, IEM2, IEM3) associated with psychosis and 85 unrelated well-known genetic diseases. This database includes an entry for each of these 100 genetic diseases. Each entry summarizes the clinical features of the respective disease (e.g. as found in OMIM or in HPO database). Each entry also includes the known gene(s) corresponding to the respective disease and under each gene all the known pathogenic/causative variants (e.g. as identified in the HGMD or ClinVar databases).

The exomes of the three above mentioned patients, "Exome 1", "Exome 2", "Exome 3", respectively, are analyzed.

In this example, existing tools (e.g. BWA, SAMTools, GATK etc.) are used to automatically analyze the patient's Exome for variation.

With regards to storage of the Exome Sequencing data, switching the FASTQ file to SAM and ultimately BAM format is useful both in terms of storage space requirements and compatibility with existing tools for analysis of the data.

Cleaning of the BAM data from pseudo-deletions and pseudo-duplications present because of sequence malalignment is helpful prior to the analysis with GATK tools which can yield the list of all variants present. Finally, having the genetic variant data available in a VCF format is helpful as this is compatible with most tools in the public domain.

Patient A in his exome ("Exome 1") has a pathogenic change from the changes listed in the "Sample database" under "Gene 1" causing IEM1 as described above. Accordingly, the other two patients have mutations in "Gene 2" (in "Exome 2") and "Gene 3" (in "Exome 3"), respectively.

The physician after the evaluation of Patient A takes advantage of the "Sample database", the encrypted genomic data of the patient (i.e. encrypted "Exome 1"), and the method provided herein. The method provided herein allows to identify all the variants in the encrypted Exome of Patient A (i.e. "Exome 1") and determines whether they are classified as pathogenic or not in the "Sample database". In this case, all the changes present in the Sample database (and so by default are pathogenic) are automatically assigned a high score (e.g. X=100) while all the remaining variants identified in the patient's Exome will be assigned a very low score (e.g. of Y=1 or 0.1).

In all three Exomes ("Exome 1", "Exome 2", "Exome 3") benign and/or pathogenic variants could be artificially assigned in some randomly selected genes corresponding to the 100 diseases listed in the "Sample database" and have the process repeated.

Searching the "Sample Database" using the keyword "Psychosis" refines the search results ranking the genetic disease in the sample database which are relevant to psychosis based on the presence of absence of a pathogenic mutation. Within these two categories the diseases will be ranked in alphabetical order.

The same process is repeated with the addition of more than one word in the original phenotype-based search (for example, phenotypes the physician identified during his evaluation: "psychosis", "dystonia", and "nystagmus"). This further refines the final ranking since in the first example the phenotype match score ("psychosis") was the same in for all diseases listed in the "Sample database" associated with psychosis but in this version their phenotype match score is adjusted based on the extent to which the clinical features entered in the search exist as part of the clinical features under the entry for the respective disease in the "Sample database".

Moreover, if the clinical features listed under each disease in the "Sample database" are assigned a different score based on the positive and negative predictive value they have for that disease (e.g. using frequencies listed in HPO database, ; during the matching process the method can also take this into consideration and refine score assignment.

In a similar application instead of using the "Sample Database" , the clinician can use ClinVar or HGMD directly within the context of the present method in order to identify the genetic variants present in the target subject's genome listed as pathogenic in the respective database and assign the score depending on the classification group each variant belongs.

### Tools

The following tools, can be useful within the context of the present disclosure:
Potentially useful curated databases include the Human Gene Mutation Database (HGMD), Diagnostic Mutation Database (DMuDB), MutaDatabase, and the ClinVar database. With regards to phenotypic data, an interface with freely available or private databases (such as OMIM, Genereviews, London Medical Databases, or Possum Web) will be needed.

In order for the search to be optimized, a standardized vocabulary of phenotypic abnormalities encountered in human disease and the semantic relationships between them will be important. The Human Phenotype Ontology (HPO), Phenomyzer (http://hum-molgen.org)/NewsGen/01-2010/000002.html), could be very useful to this end.

Mutalyzer (https://mutalyzer.nl/): The aim of this program is to support checks of sequence variant nomenclature according to the guidelines of the Human Genome Variation Society.
- OMIM (http://www.omim.org) A regularly updated catalogue of human mendelian disease focusing on the relationship between genes and their molecular variants and associated phenotypes. OMIM primarily describes single gene mendelian disorders but also includes other phenotype entries describing complex traits for which variation in a single gene results in significant contribution to the phenotype. Moreover, it includes genetic disorders shown to be due to gene deletions or duplications. To further aid in searching clinical features searches can be restricted to major anatomical headings within the Clinical Synopses.

VarSifter (http://research.nhgri.nih.gov/software/VarSifter/), freely available tool for exome analysis: "VarSifter" is a graphical Java program designed to display, sort, filter, and generally sift variation data from massively parallel sequencing experiments. It is designed to read exome-scale variation data in either a tab-delimited text file with header, or an uncompressed VCF file. These files should be pre-generated with desired annotation information one would like to view.
- Sequence Variant Analyzer, or SVA, (http://www.svaproiect.org/) is a software tool to analyze the genetic variants identified from -genome sequencing studies. SVA is designed for two specific aims: (1) To annotate the biological functions of the identified genetic variants and group them, conveniently; (2) To find the genetic variants that are associated with or responsible for the biological traits or medical outcomes of interest. SVA enables the user to take advantage of a gene filter (e.g. based on quality score, pathway, gene-ontology, OMIM information) and different biological databases when performing the functional annotation. It also allows users to explore the strength of the associations of identified variants with studied traits

Freely available statistical software like Family Based Association Test, a.k.a. FBAT, can be useful to for the calculation of the "impact score" of each variant and help support its pathogenicity, for instance, based on the cosegregation of the variant with disease.
- "UCSC Genome Browser", http://qenome.ucsc.edu/ provides for looking at the conservation of sequences across species which can be useful as a means of predicting pathogenicity.
- ANNOVAR, (http://www.openbioinformatics.org/annovar/) provides for the functional annotation of genetic variants from high-throughput sequencing data.

Still other tools include: BWA (http://bio-bwa.sourceforge.net/); SAMtools (http://samtools.sourceforge.net/); SOAP (http://soap.genomics.org.cn); Ensembl (http://www.ensembl.org/Homo_sapiens/Info/Index); DECIPHER: "Database of Chromosomal Imbalance and Phenotype in Humans Using Ensembl Resources" (http://decipher.sanger.ac.uk/); the "1000 genome database" (http://www.1000genomes.org/) a collection of all the variations in healthy individuals, "Human Genome Variation" (http://www.hgvs.org/); "Locus Specific Mutation Databases" (http://www.hgvs.org/dblist/glsdb.html): "Human Gene Mutation Database" (http://www.hgmd.cf.ac.uk/ac/); dbSNP database (http://www.ncbi.nlm.nih.gov/projects/SNP/); Genome Trax (http://www.avadisngs.com/features/genome trax); Catalogue Of Somatic Mutations In Cancer (COSMIC) database (http://www.sanger.ac.uk/perl/genetics/CGP/cosmic); Genecards (http://www.genecards.org/); PolyPhen, (http://genetics.bwh.harvard.edu/pph/); SIFT (http://sift.jcvi.org/); ANNOVAR (http://www.openbioinformatics.org/annovar/); SNPnexus (http://www.snp-nexus.org/); Mutation assersor (http://mutationassessor.org/); FASTSNP (http://fastsnp.ibms.sinica.edu.tw/pages/input CandidateGeneSearch.jsp); Conserved Domain-Based Prediction (CDPred) Software (http://research.nhgri.nih.gov/software/CDPred/); MutPred (http://mutpred.mutdb.org/); SNPtoGO (https://webtools.imbs.uk-sh.de/snptogo/); Gen2Phen (www.gen2phen.org).; A probabilistic gene network (AraNet): Integrating Rare-Variant Testing, Function Prediction, and Gene Network in Composite Resequencing-Based Genome-Wide Association Studies (CR-GWAS) (Zhu et al. 2011 Aug;1(3):233-43. PMID: 22384334); Probabilistic Disease-Gene Finder, VAAST: *A probabilistic disease-gene finder for personal genomes.* Yandell et al Genome Res. 2011 Sep;21(9):1529-42. PMID: 21700766; GeneMANIA (http://www.genemania.org); *A guide to web tools to prioritize candidate genes* (Tranchevent et al Brief Bioinform 2011; 12: 22-32); HaploReg (Compbio.mit.edu/HaploReg); Human Serum metabolome (http://www.serummetabolome.ca); *Database resources in metabolomics: an overview* (Go EP. J Neuroimmune Pharmacol 2010, 5(1):18-30); *Integration of bioinformatics resources for functional analysis of gene expression and proteomic data* (Huang et al. Front Biosci 2007, 12:5071-5088); RNA sequencing (RNA-seq) in gene expression studies: *A framework for variation discovery and genotyping using next-generation DNA sequencing data* (DePristo, M.A. et al. Nat. Genet. 43, 491-498 (2011); *Understanding mechanisms underlying human gene expression variation with RNA sequencing (* Pickrell, J.K. et al. Nature 464, 768-772 (2010); *Transcriptome genetics using second generation sequencing in a Caucasian population* (Montgomery, S.B. et al. Nature 464, 773-777 (2010). PharmaADME "Core Gene List" (http://www.pharmaadme.org/); Pharmacogenomics Knowledge Base (PharmGKB; http://www.pharmgkb.org).

Of course, the skilled artisan will understand that still other similar tools can be used within the context of the present disclosure.

### Other applications

An approach similar to the one described above for genome/exome sequencing applies to other types of genomic data analysis: one such example is Array-based comparative genomic hybridization (aCGH) technology which allows for whole-genome analysis for DNA gains and losses. In accordance with the method described, such changes in a patient with specific phenotypic features are assigned a different impact factor/weight based on pre-determined criteria similar to those used in clinical interpretation of aCGH findings. For example, many of these gains and loses are known to involve clinically important loci and lead to well known genetic syndromes so these would be assigned high impact factors. Similarly, genetic changes involving areas of the genome well conserved across-species are assigned high impact factors while deletions/duplications listed as normal variants present in the general population would be allocated minimal scores. By taking the weights of these chances into consideration, the present method aids in adjusting the phenotype-based ranking of the possible genetic conditions of the patient.

The database could also be searched using a Medical Subject Headings (MeSH)-like approach. MeSH is a comprehensive controlled vocabulary available at MEDLINE/PubMed useful for indexing journal articles and books in the life sciences which can also serve as a thesaurus that facilitates searching. Similarly, classification of clinical features in sub-groups (e.g. "nail dysplasia" or "short stature, non-skeletal dysplasia") may be helpful. A very useful such existing thesaurus is the Human Phenotype Ontology (HPO), Phenomyzer (http://hum-molgen.org/NewsGen/01-2010/000002.html). A physician may select one of these categories and obtain a list of genetic diagnoses sharing the corresponding feature. This list could be initially ranked based on different characteristics such as population prevalence. Based on the method herein, the ranking will change when integrating the patient's genomic data and this facilitates testing prioritization.

The presented approach can be used for pre-conceptual counseling, for example, in couples interested to learn about the risk for their children to develop different severe recessive diseases without necessarily learning about their own carrier status for all these diseases. By comparing the encrypted genomes of the parents for the loci corresponding to a preselected list of targeted genetic recessive diseases, only diseases for which both parents are carriers will be ranked highly above a pre-set threshold and get flagged. Hence, the couple may decide to seek counselling only for these diseases. The selection of the diseases targeted could make use of the idea for lower- and higher-risk categories or "bins" based on clinical validity and actionability. (Deploying whole genome sequencing in clinical practice and public health: meeting the challenge one bin at a time. Berg JS, Khoury MJ, Evans JP. Genet Med. 2011 Jun;13(6):499-504. PMID: 21558861).

Overall, the benefits of the present approach can be illustrated with the following analogy. We can assume that there are two different languages, Language (1) and Language (2), both using the same alphabet (e.g. Latin alphabet in French and English). Now picture someone who speaks Language (1) trying to read a book in Language (2). However, since only few words are similar enough between the two languages (e.g. between English and French) he can decipher the meaning of only a few words/phrases. The biggest part of the book remains incomprehensible. Now think of the person speaking Language (1) taking an immersion class where, through exposure, he learns more of the language (2) and can thus progressively read more and more parts of the book. The approach described hereby provides the structure for the "immersion class" that will help us progressively understand better the information encoded in the genome ("book"). Language (1) is Medical Genetics and Language (2) is Medical Genomics. Using the "alphabet" of "Medical Genetics" scientists have been able to write all the sections of the book (i.e. sequencing data of the Genome). Based on the knowledge of "Medical Genetics" we can also understand some parts of the genome (i.e. read parts of the book where words are close to the ones we already know) and associate them with genetic traits/diseases. By protecting the patients' privacy and autonomy while at the same time allowing for adequate flexibility in data analysis, the approach described facilitates the use of real patients' data (corresponding to learning by exposure in an "immersion class") as a teaching method of "Medical Genomics" (Language 2).

It should be noted that the various components and features of the embodiments described above can be combined in a variety of ways so as to provide other non-illustrated embodiments within the scope of the disclosure. As such, it is to be understood that the disclosure is not limited in its application to the details of construction and parts illustrated in the accompanying drawings and described hereinabove. The disclosure is capable of other embodiments and of being practiced in various ways. It is also to be understood that the phraseology or terminology used herein is for the purpose of description and not limitation.

## Claims

1. A computer-implemented diagnostic method of determining the genetic diseases of a subject, the method comprising:
acquiring assessed phenotypes of this subject, wherein the assessed phenotypes are selected from the group consisting of clinically assessed phenotypes, specific profiles in the transcriptome of the subject, specific profiles in the metabolome of the subject, changes in the transcriptome of the subject, changes in the metabolome of the subject;
providing a database (22) of genetic diseases linked at least in part to at least one relevant region in the genome of the subject and at least in part to at least one phenotypic characteristic;
analyzing the genome of the subject;
identifying changes in the analyzed genome by comparing it to at least one reference genome;
ranking a plurality of possible genetic diseases of this subject related to the assessed phenotypes of this subject,
**characterized in that** the ranking comprises:
- assigning weight scores to each of the identified changes based on predetermined criteria, wherein assigning weight scores to each of the identified changes based on predetermined criteria comprises a step selected from the group consisting of:
assigning a score based on the presence or absence of an identified change in a gene in the analyzed genome of the subject;
assigning a score to an identified change based on whether this identified change is known to cause a genetic disease or not;
assigning a score based on the frequency of a variant in the database (22) of genetic diseases;
running the genomic data of multiple family members of the subject and assigning a score to the identified change based on whether it segregates with a disease present in the other family members or if occurs de novo in the subject;
running the genomic data obtained from different tissues of the subject and assigning a score to the identified change based on whether it segregates with a disease present in at least one of the tissues;
assigning a score to the identified change by taking into account the strength of the association of an identified variant with a studied trait by using a statistical method to analyze the cosegregation of the identified change with a disease at a population and/or a family level, the statisitical method being selected from the group consisting of: a Bayesian analysis, an LOD score analysis, a TDT analysis and any combination thereof;
assigning a score based on the type of identified change;
assigning a score based on whether or not the identified change is present in a gene which is part of a cellular pathway already known to be involved in the pathophysiology of the assessed phenotype;
assigning a score based on the evolutionary conservation of the identified change;
assigning a score based on the pattern of inheritance within the family history of the subject; and
any combination of the foregoing;
- searching the database (22) based on the assessed phenotypes thereby providing a first ranking of possible genetic diseases, each genetic disease being related to at least one genetic change indicative of that disease;
- adjusting the first ranking of possible genetic diseases based on the assigned weight scores; and
- providing a second ranking of possible genetic diseases based on the adjustment thereby providing for determining the genetic diseases of the subject based on the second ranking.

2. A computer-implemented method according to claim 1, wherein the step of identifying is performed without providing the user access to the identified changes.

3. A computer-implemented method according to any one of claims 1 or 2, further comprising encrypting the analyzed genome prior to the step of identifying.

4. A computer-implemented method according to any one of claims 1 to 3, wherein the subject is a human subject.

5. A computer-implemented method according to any one of claims 1 to 4, wherein the genomic data of multiple family members is encrypted.

6. A computer-implemented method according to any one of claims 1 to 5, wherein the region is selected from the group consisting of a coding region, a gene, a noncoding region.

7. A computer-implemented method according to any one of claims 1 to 6, wherein the step of analyzing comprises sequencing.

8. A computer-implemented method according to any one of claims 1 to 7, wherein the change is selected from the group consisting of a mutation, a gene variant, an epigenetic change.

9. A computer-implemented method according to any one of claims 1 to 8 wherein the step of searching the database (22) comprises searching by phenotypic characteristics or by keywords related to the assessed phenotypes.

10. A diagnostic system for determining the genetic diseases of a subject, the system comprising:
a database (22) of genetic diseases linked at least in part to at least one relevant region in the genome of the genome of the subject and at least in part to at least one phenotypic characteristic;
a data storage medium (20) comprising the analyzed genome of the subject, wherein changes in the genome of the subject have been identified; and
a processor (17) comprising a user interface (16) in communication with the database (22) and the a data storage medium (20), the processor (17) providing for identifying changes in the analyzed genome, ranking a plurality of possible genetic diseases of this subject related to acquired assessed phenotypes of this subject, wherein the assessed phenotypes are selected from the group consisting of clinically assessed phenotypes, specific profiles in the transcriptome of the subject, specific profiles in the metabolome of the subject, changes in the transcriptome of the subject, changes in the metabolome of the subject,
**characterized in that** the ranking comprises:
assigning weight scores to each of the identified changes based on predetermined criteria, searching the database (22) based on the assessed phenotypes thereby providing a first ranking of possible genetic diseases, each genetic disease being related to at least one genetic change indicative of that disease and adjusting the first ranking of possible genetic diseases based on the assigned weight scores thereby providing a second ranking of possible genetic diseases based on the adjustment thereby providing for determining the genetic diseases of the subject based on the second ranking,
wherein assigning weight scores to each of the identified changes based on predetermined criteria comprises a step selected from the group consisting of:
assigning a score based on the presence or absence of an identified change in a gene in the analyzed genome of the subject;
assigning a score to an identified change based on whether this identified change is known to cause a genetic disease or not;
assigning a score based on the frequency of a variant in the database (22) of genetic diseases;
running the genomic data of multiple family members of the subject and assigning a score to the identified change based on whether it segregates with a disease present in the other family members or if occurs de novo in the subject;
running the genomic data obtained from different tissues of the subject and assigning a score to the identified change based on whether it segregates with a disease present in at least one of the tissues;
assigning a score to the identified change by taking into account the strength of the association of an identified variant with a studied trait by using a statistical method to analyze the cosegregation of the identified change with a disease at a population and/or a family level, the statistical method being selected from the group consisting of: a Bayesian analysis, an LOD score analysis, a TDT analysis and any combination thereof;
assigning a score based on the type of identified change;
assigning a score based on whether or not the identified change is present in a gene which is part of a cellular pathway already known to be involved in the pathophysiology of the assessed phenotype;
assigning a score based on the evolutionary conservation of the identified change;
assigning a score based on the pattern of inheritance within the family history of the subject; and
any combination of the foregoing.

11. A system according to claim 10, wherein the data storage medium (20) is selected from the group consisting of a CD, a DVD, a memory key, a chip, and a cloud.

12. A system according to any one of claims 10 or 11, wherein the communication between the processor (17) and the database (22) is selected from the group consisting of a local communication and a remote communication.

## Patentansprüche

1. Computerimplementiertes diagnostisches Verfahren zum Bestimmen der genetischen Erkrankungen eines Subjekts, wobei das Verfahren umfasst:
Erfassen von bewerteten Phänotypen dieses Subjekts, wobei die bewerteten Phänotypen aus der Gruppe ausgewählt werden, die aus klinisch bewerteten Phänotypen, spezifischen Profilen im Transkriptom des Subjekts, spezifischen Profilen im Metabolom des Subjekts, Veränderungen im Transkriptom des Subjekts, Veränderungen im Metabolom des Subjekts besteht:
Bereitstellung einer Datenbank (22) genetisch bedingter Krankheiten, die mindestens teilweise mit mindestens einer relevanten Region im Genom des Subjekts und mindestens teilweise mit mindestens einem phänotypischen Merkmal verbunden sind;
Analyse des Genoms des Subjekts;
Identifizieren von Veränderungen im analysierten Genom durch Vergleichen mit mindestens einem Referenzgenom;
Einstufung mehrerer möglicher genetischer Erkrankungen dieses Subjekts in Bezug auf die bewerteten Phänotypen dieses Subjekts,
**dadurch gekennzeichnet, dass** die Einstufung umfasst:
- Zuweisen von Gewichtsbewertungen zu jeder der identifizierten Veränderungen auf Basis vorgegebener Kriterien, wobei das Zuweisen von Gewichtsbewertungen zu jeder der identifizierten Veränderungen auf Basis vorgegebener Kriterien einen Schritt umfasst, der aus der Gruppe ausgewählt ist, die besteht aus:
Zuweisen einer Bewertung auf Basis des Vorhandenseins oder Fehlens einer identifizierten Veränderung in einem Gen im analysierten Genom des Subjekts;
Zuweisen einer Bewertung zu einer identifizierten Veränderung basierend darauf, ob bekannt ist, dass diese identifizierte Veränderung eine genetische Krankheit verursacht oder nicht;
Zuweisen einer Bewertung basierend auf der Häufigkeit einer Variante in der Datenbank (22) genetisch bedingter Krankheiten;
Berechnen der Genomdaten mehrerer Familienmitglieder des Subjekts und Zuweisen einer Bewertung zu der identifizierten Veränderung basierend darauf, ob sie mit einer Krankheit segregiert, die bei den anderen Familienmitgliedern vorhanden ist, oder ob sie de novo bei dem Subjekt auftritt;
Berechnen der Genomdaten, die aus verschiedenen Geweben des Subjekts erhalten wurden, und Zuweisen einer Bewertung zu der identifizierten Veränderung basierend darauf, ob sie mit einer in mindestens einem der Gewebe vorhandenen Krankheit segregiert;
Zuweisen einer Bewertung zu der identifizierten Veränderung unter Berücksichtigung der Stärke der Assoziation einer identifizierten Variante mit einem untersuchten Merkmal unter Verwendung einer statistischen Methode zur Analyse der Cosegregation der identifizierten Veränderung mit einer Krankheit auf Bevölkerungs- und/oder Familienebene, wobei die statistische Methode ausgewählt wird aus der Gruppe bestehend aus: einer Bayes'schen Analyse, einer LOD-Score-Analyse, einer TDT-Analyse und einer beliebigen Kombination davon;
Zuweisen einer Punktzahl basierend auf der Art der identifizierten Veränderung;
Zuweisen eines Scores basierend darauf, ob die identifizierte Veränderung in einem Gen vorhanden ist oder nicht, das Teil eines Zellwegs ist, von dem bereits bekannt ist, dass er an der Pathophysiologie des bewerteten Phänotyps beteiligt ist;
Zuweisen einer Punktzahl basierend auf der evolutionären Erhaltung der identifizierten Veränderung;
Zuweisen einer Punktzahl basierend auf dem Vererbungsmuster innerhalb der Familiengeschichte des Subjekts; und
jeder Kombination des Vorstehenden;
- Durchsuchen der Datenbank (22) auf der Grundlage der bewerteten Phänotypen, wodurch eine erste Einstufung möglicher genetischer Krankheiten bereitgestellt wird, wobei jede genetische Krankheit mit mindestens einer genetischen Veränderung zusammenhängt, die auf diese Krankheit hinweist;
- Anpassen der ersten Einstufung möglicher genetischer Krankheiten auf Basis der zugewiesenen Gewichtsbewertungen; und
- Bereitstellen einer zweiten Einstufung möglicher genetischer Krankheiten auf Basis der Anpassung, wodurch das Bestimmen der genetischen Krankheiten des Subjekts basierend auf der zweiten Einstufung bereitgestellt wird.

2. Computerimplementiertes Verfahren nach Anspruch 1, wobei der Schritt des Identifizierens ausgeführt wird, ohne dem Benutzer Zugriff auf die identifizierten Veränderungen zu gewähren.

3. Computerimplementiertes Verfahren nach einem der Ansprüche 1 oder 2, ferner umfassend das Verschlüsseln des analysierten Genoms vor dem Schritt des Identifizierens.

4. Computerimplementiertes Verfahren nach einem der Ansprüche 1 bis 3, wobei das Subjekt eine Person ist.

5. Computerimplementiertes Verfahren nach einem der Ansprüche 1 bis 4, wobei die Genomdaten mehrerer Familienmitglieder verschlüsselt sind.

6. Computerimplementiertes Verfahren nach einem der Ansprüche 1 bis 5, wobei die Region ausgewählt ist aus der Gruppe bestehend aus einer codierenden Region, einem Gen, einer nicht codierenden Region.

7. Computerimplementiertes Verfahren nach einem der Ansprüche 1 bis 6, wobei der Schritt des Analysierens das Sequenzieren umfasst.

8. Computerimplementiertes Verfahren nach einem der Ansprüche 1 bis 7, wobei die Veränderung ausgewählt ist aus der Gruppe bestehend aus einer Mutation, einer Genvariante, einer epigenetischen Veränderung.

9. Computerimplementiertes Verfahren nach einem der Ansprüche 1 bis 8, wobei der Schritt des Durchsuchens der Datenbank (22) das Suchen nach phänotypischen Merkmalen oder nach Schlüsselwörtern umfasst, die sich auf die bewerteten Phänotypen beziehen.

10. Diagnosesystem zum Bestimmen der genetischen Erkrankungen eines Subjekts, wobei das System umfasst:
eine Datenbank (22) genetisch bedingter Krankheiten, die mindestens teilweise mit mindestens einer relevanten Region im Genom des Genoms des Subjekts und mindestens teilweise mit mindestens einem phänotypischen Merkmal verknüpft sind;
ein Datenspeichermedium (20), das das analysierte Genom des Subjekts umfasst, wobei Veränderungen im Genom des Subjekts identifiziert wurden; und
einen Prozessor (17), der eine Benutzerschnittstelle (16) in Kommunikation mit der Datenbank (22) und einem Datenspeichermedium (20) umfasst, wobei der Prozessor (17) das Identifizieren von Veränderungen im analysierten Genom vorsieht, wobei mehrere mögliche genetische Krankheiten dieses Subjekts in Bezug auf erworbene bewertete Phänotypen dieses Subjekts eingestuft werden, wobei die bewerteten Phänotypen aus der Gruppe ausgewählt sind, die aus klinisch bewerteten Phänotypen, spezifischen Profilen im Transkriptom des Subjekts, spezifischen Profilen im Metabolom des Subjekts, Veränderungen im Transkriptom des Subjekts, Veränderungen im Metabolom des Subjekts besteht,
**dadurch gekennzeichnet, dass** die Einstufung umfasst:
Zuweisen von Gewichtsbewertungen zu jeder der identifizierten Veränderungen Basis vorgegebener Kriterien, Durchsuchen der Datenbank (22) auf Basis bewerteter Phänotypen, wodurch eine erste Einstufung möglicher genetischer Krankheiten bereitgestellt wird, wobei jede genetische Krankheit mit mindestens einer genetischen Veränderung zusammenhängt, die auf diese Krankheit hinweist und Anpassen der ersten Einstufung möglicher genetischer Krankheiten auf Basis der zugewiesenen Gewichtsbewertungen, wodurch eine zweite Einstufung möglicher genetischer Krankheiten auf Basis der Anpassung bereitgestellt wird, wodurch die Bestimmung der genetischen Krankheiten des Subjekts auf Basis der zweiten Einstufung bereitgestellt wird;
wobei das Zuweisen von Gewichtsbewertungen zu jeder der identifizierten Veränderungen auf Basis vorgegebener Kriterien einen Schritt umfasst, der aus der Gruppe ausgewählt ist, die besteht aus:
Zuweisen einer Bewertung auf Basis des Vorhandenseins oder Fehlens einer identifizierten Veränderung in einem Gen im analysierten Genom des Subjekts;
Zuweisen einer Bewertung zu einer identifizierten Veränderung basierend darauf, ob bekannt ist, dass diese identifizierte Veränderung eine genetische Krankheit verursacht oder nicht;
Zuweisen einer Bewertung basierend auf der Häufigkeit einer Variante in der Datenbank (22) genetisch bedingter Krankheiten;
Berechnen der Genomdaten mehrerer Familienmitglieder des Subjekts und Zuweisen einer Bewertung zu der identifizierten Veränderung basierend darauf, ob sie mit einer Krankheit segregiert, die bei den anderen Familienmitgliedern vorhanden ist, oder ob sie de novo bei dem Subjekt auftritt;
Berechnen der Genomdaten, die aus verschiedenen Geweben des Subjekts erhalten wurden, und Zuweisen einer Bewertung zu der identifizierten Veränderung basierend darauf, ob sie mit einer in mindestens einem der Gewebe vorhandenen Krankheit segregiert;
Zuweisen einer Bewertung zu der identifizierten Veränderung unter Berücksichtigung der Stärke der Assoziation einer identifizierten Variante mit einem untersuchten Merkmal unter Verwendung einer statistischen Methode zur Analyse der Cosegregation der identifizierten Veränderung mit einer Krankheit auf Bevölkerungs- und/oder Familienebene, wobei die statistische Methode ausgewählt wird aus der Gruppe bestehend aus: einer Bayes'schen Analyse, einer LOD-Score-Analyse, einer TDT-Analyse und einer beliebigen Kombination davon;
Zuweisen einer Punktzahl basierend auf der Art der identifizierten Veränderung;
Zuweisen eines Scores basierend darauf, ob die identifizierte Veränderung in einem Gen vorhanden ist oder nicht, das Teil eines Zellwegs ist, von dem bereits bekannt ist, dass er an der Pathophysiologie des bewerteten Phänotyps beteiligt ist;
Zuweisen einer Punktzahl basierend auf der evolutionären Erhaltung der identifizierten Veränderung;
Zuweisen einer Punktzahl basierend auf dem Vererbungsmuster innerhalb der Familiengeschichte des Subjekts; und
eine beliebige Kombination der Vorstehenden.

11. System nach Anspruch 10, wobei das Datenspeichermedium (20) ausgewählt ist aus der Gruppe bestehend aus einer CD, einer DVD, einem Speicherschlüssel, einem Chip und einer Wolke.

12. System nach einem der Ansprüche 10 oder 11, wobei die Kommunikation zwischen dem Prozessor (17) und der Datenbank (22) aus der Gruppe ausgewählt ist, die aus einer lokalen Kommunikation und einer Fernkommunikation besteht.

## Revendications

1. Procédé de diagnostic mis en œuvre par ordinateur pour déterminer les maladies génétiques d'un sujet, le procédé comprenant :
l'acquisition de phénotypes évalués de ce sujet, les phénotypes évalués étant choisis dans le groupe constitué de phénotypes évalués cliniquement, de profils spécifiques dans le transcriptome du sujet, de profils spécifiques dans le métabolome du sujet, de changements dans le transcriptome du sujet, de changements dans le métabolome du sujet ;
le fait de se munir d'une base de données (22) de maladies génétiques liées au moins en partie à au moins une région pertinente du génome du sujet et au moins en partie à au moins une caractéristique phénotypique ;
l'analyse du génome du sujet ;
l'identification de changements dans le génome analysé en le comparant à au moins un génome de référence ;
le classement d'une pluralité de maladies génétiques possibles de ce sujet en lien avec les phénotypes évalués de ce sujet,
**caractérisé en ce que** le classement comprend :
- l'attribution de scores de pondération à chacun des changements identifiés sur la base de critères prédéterminés, dans lequel l'attribution de scores de pondération à chacun des changements identifiés sur la base de critères prédéterminés comprend une étape choisie dans le groupe constitué de :
l'attribution d'un score basé sur la présence ou l'absence d'un changement identifié d'un gène dans le génome analysé du sujet ;
l'attribution d'un score à un changement identifié selon que ce changement identifié est connu pour provoquer une maladie génétique ou non ;
l'attribution d'un score basé sur la fréquence d'une variante dans la base de données (22) des maladies génétiques ;
l'examen des données génomiques de plusieurs membres de la famille du sujet et l'attribution d'un score au changement identifié selon qu'il est trié avec une maladie présente chez les autres membres de la famille ou qu'il se produit de novo chez le sujet ;
l'examen des données génomiques obtenues à partir de différents tissus du sujet et l'attribution d'un score au changement identifié selon qu'il est trié avec une maladie présente dans au moins l'un des tissus ;
l'attribution d'un score au changement identifié en tenant compte de la force de l'association d'une variante identifiée avec une caractéristique étudiée en utilisant une méthode statistique pour analyser le tri commun du changement identifié avec une maladie au niveau d'une population et/ou d'une famille, la méthode statistique étant choisie dans le groupe constitué par : une analyse bayésienne, une analyse de score LOD, une analyse TDT et toute combinaison de celles-ci ;
l'attribution d'un score en fonction du type de changement identifié ;
l'attribution d'un score basé sur la présence ou non du changement identifié dans un gène qui fait partie d'une voie cellulaire déjà connue pour être impliquée dans la physiopathologie du phénotype évalué ;
l'attribution d'un score basé sur la conservation évolutive du changement identifié ;
l'attribution d'un score basé sur le schéma d'héritage dans l'histoire familiale du sujet ; et
toute combinaison de ce qui précède ;
- la recherche dans la base de données (22) sur la base des phénotypes évalués, fournissant ainsi un premier classement des maladies génétiques possibles, chaque maladie génétique étant liée à au moins un changement génétique indicatif de cette maladie ;
- l'ajustement du premier classement des maladies génétiques possibles sur la base des scores de pondération attribués ; et
- le fait de se munir d'un second classement des maladies génétiques possibles sur la base de l'ajustement, permettant ainsi de déterminer les maladies génétiques du sujet sur la base du second classement.

2. Procédé mis en œuvre par ordinateur selon la revendication 1, dans lequel l'étape d'identification est exécutée sans fournir à l'utilisateur l'accès aux modifications identifiées.

3. Procédé mis en œuvre par ordinateur selon l'une quelconque des revendications 1 ou 2, comprenant en outre le cryptage du génome analysé avant l'étape d'identification.

4. Procédé mis en œuvre par ordinateur selon l'une quelconque des revendications 1 à 3, dans lequel le sujet est un sujet humain.

5. Procédé mis en œuvre par ordinateur selon l'une quelconque des revendications 1 à 4, dans lequel les données génomiques de plusieurs membres de la famille sont cryptées.

6. Procédé mis en œuvre par ordinateur selon l'une quelconque des revendications 1 à 5, dans lequel la région est choisie dans le groupe constitué d'une région codante, d'un gène, d'une région non codante.

7. Procédé mis en œuvre par ordinateur selon l'une quelconque des revendications 1 à 6, dans lequel l'étape d'analyse comprend le séquençage.

8. Procédé mis en œuvre par ordinateur selon l'une quelconque des revendications 1 à 7, dans lequel le changement est choisi dans le groupe constitué d'une mutation, d'une variante de gène, d'un changement épigénétique.

9. Procédé mis en œuvre par ordinateur selon l'une quelconque des revendications 1 à 8, dans lequel l'étape de recherche dans la base de données (22) comprend une recherche par caractéristiques phénotypiques ou par mots clés liés aux phénotypes évalués.

10. Système de diagnostic pour déterminer les maladies génétiques d'un sujet, le système comprenant :
une base de données (22) de maladies génétiques liées au moins en partie à au moins une région pertinente du génome du sujet et au moins en partie à au moins une caractéristique phénotypique ;
un support de stockage de données (20) comprenant le génome analysé du sujet, dans lequel des changements dans le génome du sujet ont été identifiés ; et
un processeur (17) comprenant une interface utilisateur (16) en communication avec la base de données (22) et le support de stockage de données (20), le processeur (17) permettant d'identifier les changements dans le génome analysé, classant une pluralité de maladies génétiques possibles de ce sujet liées à des phénotypes évalués acquis de ce sujet, dans lequel les phénotypes évalués sont choisis dans le groupe constitué de phénotypes évalués cliniquement, de profils spécifiques dans le transcriptome du sujet, de profils spécifiques dans le métabolome du sujet, de changements dans le transcriptome du sujet, de changements dans le métabolome du sujet,
**caractérisé en ce que** le classement comprend :
l'attribution de scores de pondération à chacun des changements identifiés sur la base de critères prédéterminés, la recherche dans la base de données (22) sur la base des phénotypes évalués, fournissant ainsi un premier classement des maladies génétiques possibles, chaque maladie génétique étant liée à au moins un changement génétique indicatif de cette maladie et l'ajustement du premier classement des maladies génétiques possibles sur la base des scores de pondération attribués, fournissant ainsi un second classement des maladies génétiques possibles sur la base de l'ajustement, permettant ainsi de déterminer les maladies génétiques du sujet sur la base du second classement,
dans lequel l'attribution de scores de pondération à chacun des changements identifiés sur la base de critères prédéterminés comprend une étape choisie dans le groupe constitué par :
l'attribution d'un score basé sur la présence ou l'absence d'un changement identifié d'un gène dans le génome analysé du sujet ;
l'attribution d'un score à un changement identifié selon que ce changement identifié est connu pour provoquer une maladie génétique ou non ;
l'attribution d'un score basé sur la fréquence d'une variante dans la base de données (22) des maladies génétiques ;
l'examen des données génomiques de plusieurs membres de la famille du sujet et l'attribution d'un score au changement identifié selon qu'il est trié avec une maladie présente chez les autres membres de la famille ou qu'il se produit de novo chez le sujet ;
l'examen des données génomiques obtenues à partir de différents tissus du sujet et l'attribution d'un score au changement identifié selon qu'il est trié avec une maladie présente dans au moins l'un des tissus ;
l'attribution d'un score au changement identifié en tenant compte de la force de l'association d'une variante identifiée avec une caractéristique étudiée en utilisant une méthode statistique pour analyser le tri commun du changement identifié avec une maladie au niveau d'une population et/ou d'une famille, la méthode statistique étant choisie dans le groupe constitué par : une analyse bayésienne, une analyse de score LOD, une analyse TDT et toute combinaison de celles-ci ;
l'attribution d'un score en fonction du type de changement identifié ;
l'attribution d'un score basé sur la présence ou non du changement identifié dans un gène qui fait partie d'une voie cellulaire déjà connue pour être impliquée dans la physiopathologie du phénotype évalué ;
l'attribution d'un score basé sur la conservation évolutive du changement identifié ;
l'attribution d'un score basé sur le schéma d'héritage dans l'histoire familiale du sujet ; et
toute combinaison de ce qui précède.

11. Système selon la revendication 10, dans lequel le support de stockage de données (20) est choisi dans le groupe constitué d'un CD, d'un DVD, d'une clé USB, d'une puce et d'un cloud.

12. Système selon l'une quelconque des revendications 10 ou 11, dans lequel la communication entre le processeur (17) et la base de données (22) est choisie dans le groupe constitué d'une communication locale et d'une communication distante.
